# EUROPEAN PATENT SPECIFICATION

(11) **EP 4 561 678 B1**
(45) Date of publication and mention of the grant of the patent: **18.11.2026**
(21) Application number: 23757759.8
(22) Date of filing: 27.07.2023
(51) Int. Cl.: A61N 1/36, A61B 5/311, A61B 5/388, A61B 5/00, A61N 1/372

(54) **AUTOMATED PROGRAMMING BASED ON ECAP SIGNALS**
AUTOMATISCHE PROGRAMMIERUNG AUF DER BASIS VON ECAP-SIGNALEN
PROGRAMMATION AUTOMATISÉE BASÉE SUR DES SIGNAUX ECAP

(30) Priority: 27.07.2022 US 202263392799 P
(43) Date of publication of application: 04.06.2025
(73) Proprietor: Medtronic, Inc., Minneapolis, Minnesota 55432 (US)
(72) Inventor: MUELLER, Jerel K., Minneapolis, Minnesota 55432 (US); SCHMELING, Andrew L., Holmen, Wisconsin 54636 (US); SIMONS, David W., Minneapolis, Minnesota 55432 (US); BORNHOFT, Reid K., Minneapolis, Minnesota 55432 (US); STRAKA, Malgorzata M., Minneapolis, Minnesota 55432 (US); NEDRUD, Joshua J., Minneapolis, Minnesota 55432 (US); CLELAND, Andrew J., Minneapolis, Minnesota 55432 (US); LITVAK, Leonid M., 46733 Herzliya (IL); SKERKER, Abigail Lauren, Minneapolis, Minnesota 55432 (US); KHARAM, Aleksandra P., Minneapolis, Minnesota 55432 (US)
(74) Representative: Zimmermann & Partner Patentanwälte mbB
(86) International application number: PCT/US2023/028867
(87) International publication number: WO 2024/026031

(56) References cited:
- WO-A1-2022/150638
- WO-A1-2022/182611
- US-A1- 2014 236 257
- US-A1- 2020 316 391
- US-A1- 2021 187 297

## Description

This application is a PCT application claiming priority to and the benefit of U.S. Provisional Patent Application No. 63/392,799, filed July 27, 2022.

### TECHNICAL FIELD

This disclosure generally relates to programming electrical simulation, and more specifically, selecting parameter values based on a physiological parameter.

### BACKGROUND

Medical devices may be external or implanted and may be used to deliver electrical stimulation therapy to patients via various tissue sites to treat a variety of symptoms or conditions such as chronic pain, tremor, Parkinson's disease, epilepsy, urinary or fecal incontinence, sexual dysfunction, obesity, or gastroparesis. A medical device may deliver electrical stimulation therapy via one or more leads that include electrodes located proximate to target locations associated with the brain, the spinal cord, pelvic nerves, peripheral nerves, or the gastrointestinal tract of a patient. Stimulation proximate the spinal cord, proximate the sacral nerve, within the brain, and proximate peripheral nerves are often referred to as spinal cord stimulation (SCS), sacral neuromodulation (SNM), deep brain stimulation (DBS), and peripheral nerve stimulation (PNS), respectively.

Electrical stimulation may be delivered to a patient by the medical device in a train of electrical pulses, and parameters of the electrical pulses may include a frequency, an amplitude, a pulse width, and a pulse shape. An evoked compound action potential (ECAP) is synchronous firing of a population of neurons which occurs in response to the application of a stimulus including, in some cases, an electrical stimulus by a medical device. The ECAP may be detectable as being a separate event from the stimulus itself, and the ECAP may reveal characteristics of the effect of the stimulus on the nerve fibers. Documents WO 2022/150638 A1, US 2020/316391 A1 and US 2014/236257 A1 relate to medical stimulation and/or sensing devices.

### SUMMARY

The invention is defined in the appended claims and relates to a system and a computer-readable medium. A method described herein is useful to understand the invention. In general, systems, devices, and techniques are described for analyzing evoked compound action potential (ECAP) signals and determining an ECAP threshold of a patient using the analyzed ECAP signals. A system may determine one or more stimulation parameter values for subsequent electrical stimulation based on the ECAP threshold.

Devices and systems described herein may determine ECAP characteristic values from received ECAP signals and generate at least a portion of a growth curve of the ECAP characteristic values. The ECAP signals may be sensed in response to the delivery of respective stimulation pulses. In some examples, a user may provide a maximum amplitude value for the stimulation pulses delivered to elicit the respective ECAP signals. A growth curve may be a curve indicating the relationship of ECAP characteristic values to a stimulation parameter, such as amplitude or pulse width. An IMD or programmer may determine a the ECAP threshold based on the ECAP characteristic values and determine (e.g., create or adjust) one or more parameter values that define subsequent electrical stimulation. In some examples, the system may generate growth curves and corresponding ECAP thresholds for multiple electrode combinations. The system may display the growth curves or ECAP thresholds on a user interface to inform user selection of one of the electrode combination for subsequent therapy and/or automatically select the electrode combination for subsequent stimulation therapy.

In one example, this disclosure describes a system that includes processing circuitry configured to: receive a maximum amplitude value for a plurality of stimulation pulses; control stimulation generation circuitry to begin delivery of the plurality of stimulation pulses at an initial amplitude value less than the maximum amplitude value and iteratively increase a subsequent amplitude value of a next stimulation pulse of the plurality of stimulation pulses up to the maximum amplitude value; receive, for each stimulation pulse of the plurality of stimulation pulses, evoked compound action potential (ECAP) signal information, wherein the ECAP signal information comprises ECAP signals sensed by sensing circuitry; determine, from the ECAP signal information for each stimulation pulse, ECAP characteristic values for each of the ECAP signals; and determine, based on the ECAP characteristic values and during the delivery of the stimulation pulses with iteratively increasing amplitude values, at least one threshold from which one or more stimulation parameter values for subsequent stimulation pulses are determined.

In another example, this disclosure describes a method including: receiving, by processing circuitry, a maximum amplitude value for a plurality of stimulation pulses; controlling, by the processing circuitry, stimulation generation circuitry to begin delivery of the plurality of stimulation pulses at an initial amplitude value less than the maximum amplitude value and iteratively increase a subsequent amplitude value of a next stimulation pulse of the plurality of stimulation pulses up to the maximum amplitude value; receive, by the processing circuitry and for each stimulation pulse of the plurality of stimulation pulses, evoked compound action potential (ECAP) signal information, wherein the ECAP signal information comprises ECAP signals sensed by sensing circuitry; determining, by the processing circuitry and from the ECAP signal information for each stimulation pulse, ECAP characteristic values for each of the ECAP signals; and determining, by the processing circuitry and based on the ECAP characteristic values and during the delivery of the stimulation pulses with iteratively increasing amplitude values, at least one threshold from which one or more stimulation parameter values for subsequent stimulation pulses are determined.

In another example, this disclosure describes a computer-readable medium including instructions that, when executed, control processing circuitry to: receive a maximum amplitude value for a plurality of stimulation pulses; control stimulation generation circuitry to begin delivery of the plurality of stimulation pulses at an initial amplitude value less than the maximum amplitude value and iteratively increase a subsequent amplitude value of a next stimulation pulse of the plurality of stimulation pulses up to the maximum amplitude value; receive, for each stimulation pulse of the plurality of stimulation pulses, evoked compound action potential (ECAP) signal information, wherein the ECAP signal information comprises ECAP signals sensed by sensing circuitry; determine, from the ECAP signal information for each stimulation pulse, ECAP characteristic values for each of the ECAP signals; and determine, based on the ECAP characteristic values and during the delivery of the stimulation pulses with iteratively increasing amplitude values, at least one threshold from which one or more stimulation parameter values for subsequent stimulation pulses are determined.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF DRAWINGS

FIG. 1 is a conceptual diagram illustrating an example system that includes an implantable medical device (IMD) configured to deliver spinal cord stimulation (SCS) therapy and an external programmer.
FIG. 2 is a block diagram illustrating an example configuration of components of the IMD of FIG. 1.
FIG. 3 is a block diagram illustrating an example configuration of components of an example external programmer.
FIG. 4 is a graph of example evoked compound action potentials (ECAPs) sensed for respective stimulation pulses.
FIG. 5 is an example growth curve of characteristic values for sensed ECAPs.
FIG. 6A is an example user interface configured to receive user input and present information for determining a threshold from ECAP signals.
FIG. 6B is an example user interface configured to receive user input selecting a ramp rate for stimulation pulse delivery during a sweep of amplitudes.
FIG. 7 is an example growth curve of ECAP characteristic values and real-time status of the threshold based on available ECAP characteristics as stimulation pulses are delivered.
FIG. 8A is an example user interface configured to receive user input and present information regarding sensed ECAP signals and determined ECAP characteristics.
FIG. 8B is an example user interface configured to receive user input and present information regarding sensed ECAP signals and growth curves for multiple electrode combinations.
FIG. 8C is an example user interface configured to present a summary of determined thresholds and recommendations based on received ECAP signals for multiple electrode combinations.
FIG. 9 is a flow diagram illustrating an example technique for determining a threshold.
FIG. 10 is a flow diagram illustrating an example technique for determining a threshold for one or more electrode combinations through a sweep of pulses.
FIG. 11 is a flow diagram illustrating an example technique for determining an ECAP threshold and controlling electrical stimulation.

### DETAILED DESCRIPTION

The disclosure describes examples of medical devices, systems, and techniques for analyzing evoked compound action potential (ECAP) signals and determining one or more thresholds (e.g., an ECAP threshold) that is leveraged for selecting one or more stimulation parameter values of subsequent stimulation. Electrical stimulation therapy is typically delivered to a target tissue (e.g., nerves of the spinal cord or muscle) of a patient via two or more electrodes. Parameters of the electrical stimulation therapy (e.g., electrode combination, voltage or current amplitude, pulse width, pulse frequency, etc.) are selected by a clinician and/or the patient to provide relief from various symptoms, such as pain, systems from nervous system disorders, symptoms from muscle disorders, etc. Various thresholds, such as a perception threshold and/or discomfort threshold, associated with patient feedback on stimulation may be determined for the patient and used to select and/or recommend parameters of the stimulation therapy.

ECAPs are a measure of neural recruitment, because each ECAP signal represents the superposition of electrical potentials generated from a population of axons firing in response to an electrical stimulus (e.g., a stimulation pulse). Changes in a characteristic (e.g., an amplitude of a portion of the signal or area under the curve of the signal) of an ECAP signal occurs as a function of how many axons have been activated by the delivered stimulation pulse. For a given set of parameter values that define the stimulation pulse and a given distance between the electrodes and target nerve, the detected ECAP signal may have a certain characteristic value (e.g., amplitude).

In some examples, effective stimulation therapy may rely on a certain level of neural recruitment at a target nerve. This effective stimulation therapy may provide relief from one or more conditions (e.g., patient perceived pain) without an unacceptable level of side effects (e.g., overwhelming perception of stimulation). Manually identifying one or more patient thresholds from ECAP signals, such as a perception threshold, can be time-consuming for the clinician and rely on subjective feedback from the patient. Therefore, since clinicians may have time pressure when setting up stimulation and patients may not yet be accustomed to how stimulation can be perceived, the resulting manually derived perception thresholds may be inaccurate, initial stimulation therapy may be less effective than possible. Patients may thus need to return to the clinic in order to update the thresholds and/or parameter values defining stimulation therapy. These issues may increase the time and resources needed achieve therapy efficacy possible with the therapy system or reduce the likelihood that the patient receives efficacious therapy.

As described herein, systems, devices, and techniques are described for analyzing ECAP signals sensed from the patient in order to determine one or more characteristic values of the ECAP signals, and determining one or more ECAP thresholds (e.g., an estimated neural threshold) from the ECAP characteristic values. In one example, the system may calculate the absolute value of the difference between two adjacent peaks in the ECAP signal (e.g., between a negative peak and a positive peak, such as the N1 and P2 peaks) to determine an ECAP characteristic value from each ECAP signal from respective stimulation pulses having different parameter values and then automatically determine an ECAP threshold in real-time as the ECAP signals are received from subsequent stimulation pulses. In this manner, the system can stop delivering additional stimulation pulses during a sweep of different parameter values (such as increasing the amplitude in subsequent stimulation pulses) as soon as the system has sufficient ECAP characteristic value data to determine an ECAP threshold. This can decrease initial setup time and reduce the likelihood that the patient perceives these stimulation pulses or perceives any stimulation pulses having a anything greater than a relatively low intensity.

The IMD may utilize the characteristic values of the ECAP signals to determine an ECAP threshold automatically (e.g., without patient feedback indicating the sensations felt during stimulation). This ECAP threshold may be referred to an estimated neural threshold in some examples, and may be similar to a perception threshold that the patient may determine by providing subjective patient feedback on the lowest intensity of stimulation that can be perceived by the patient. For example, the IMD (or another device, such as an external programmer or other external computing device) may estimate a neural threshold for the patient based on at least a portion of a curve of ECAP characteristic values determined from ECAP signals elicited by respective stimulation pulses of a sweep of pulses defined by different values for one or more stimulation parameter values (e.g., a sweep of pulses having incrementally increasing parameter values such as amplitude). The IMD may update the curve in real-time by adding the ECAP characteristic value to the curve after each ECAP signal is received from the respective stimulation pulse delivered as part of the sweep of pulses. As soon as the IMD determines an inflection point in the curve or otherwise determines that the ECAP characteristic values start to increase with increasing parameter value, the IMD can determine the ECAP threshold as the ECAP characteristic value or parameter value associated with that identified point in the curve.

In some examples, the system may use one or more user inputs to constrain the sweep of pulses delivered to the patient as part of the initial system setup (e.g., calibration) and determination of the ECAP threshold. For example, the system may receive user input setting a maximum parameter value (e.g., amplitude or pulse width) that the system uses to set the maximum value for the parameter that is being adjusted for the pulses that are part of the sweep of pulses. The system may present a representation of this maximum parameter value on a display to indicate how the maximum parameter value is set with respect to the parameter values of currently delivered stimulation pulses. In some examples, the system may perform the sweep of pulses on multiple different electrode combinations, where the electrode combinations are selected by the system or in response to user input requesting the electrode combinations. The sweep of pulses on each electrode combination may be performed separately (e.g., one sweep per electrode combination at a time) or concurrently (e.g., interleaving pulses of the sweep for each electrode combination together). The system may then determine ECAP thresholds for each electrode combination and display the ECAP thresholds for each electrode combination and/or recommend an electrode combination based on the ECAP thresholds. The system may then receive user input selecting the electrode combination to be used for subsequent stimulation therapy.

The ECAP threshold, e.g., the estimated neural threshold, may be similar to the perception threshold for the patient. The IMD may use this estimated neural threshold to set initial stimulation amplitudes and/or set one or more feedback variables (e.g., thresholds or targets) to which subsequent ECAP characteristic values are compared for feedback that informs one or more aspects of electrical stimulation during closed-loop stimulation, such as intensity of subsequent electrical stimulation therapy. For example, the IMD may adjust one or more parameter values that defines subsequent electrical stimulation based on the characteristic value and the estimated neural threshold. The IMD may monitor the characteristic values from respective ECAP signals over time and increase or decrease parameter values in order to maintain a target characteristic value or range of values, which may be based on the estimated neural threshold. In another example, the IMD may monitor the characteristic values from ECAP signals over time and reduce a stimulation parameter value when the characteristic value exceeds a threshold in order to reduce the likelihood of overstimulation as perceived by the patient. The IMD may employ these or other control policies based on the determined characteristic value from sensed ECAP signals.

In some examples, the ECAPs detected by an IMD may be ECAPs elicited by stimulation pulses intended to contribute to therapy of a patient or separate pulses (e.g., control pulses) configured to elicit ECAPs that are detectable by the IMD. Nerve impulses detectable as the ECAP signal travel quickly along the nerve fiber after the delivered stimulation pulse first depolarizes the nerve. If the stimulation pulse delivered by first electrodes has a pulse width that is too long, or the stimulation pulses have a pulse frequency to high, different electrodes configured to sense the ECAP will sense the stimulation pulse itself or the next stimulation pulse as an artifact (e.g., detection of delivered charge itself as opposed to detection of a physiological response to the delivered stimulus) that obscures the lower amplitude ECAP signal. However, the ECAP signal loses fidelity as the electrical potentials propagate from the electrical stimulus, because different nerve fibers propagate electrical potentials at different speeds, and fibers in the spine contributing to the ECAP are pruned off. Therefore, sensing the ECAP at a long distance from the stimulating electrodes may help avoid the artifact caused by a stimulation pulse with a long pulse width, but the ECAP signal may be too small or lose fidelity needed to detect changes to the ECAP signal that occur when the electrode-to-target-tissue distance changes. In other words, the system may not be able to identify, at any distance from the stimulation electrodes, ECAPs from stimulation pulses configured to provide a therapy to the patient. Therefore, the system may use a control pulse configured to elicit ECAP signals in certain situations. The control pulse may or may not contribute to a therapeutic benefit to the patient.

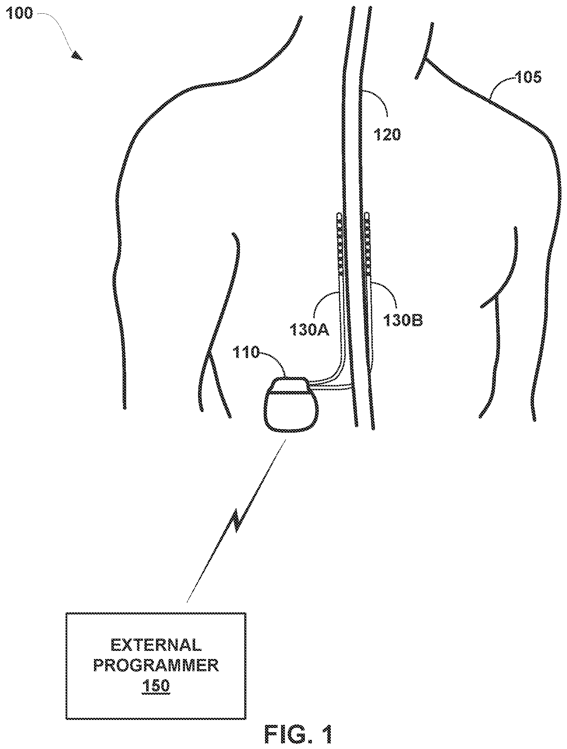

FIG. 1 is a conceptual diagram illustrating an example system 100 that includes an implantable medical device (IMD) 110 configured to deliver spinal cord stimulation (SCS) therapy, and an external programmer 150. Although the techniques described in this disclosure are generally applicable to a variety of medical devices including external devices and IMDs, application of such techniques to IMDs and, more particularly, implantable electrical stimulators (e.g., neurostimulators) will be described for purposes of illustration. More particularly, the disclosure will refer to an implantable SCS system for purposes of illustration, but without limitation as to other types of medical devices or other therapeutic applications of medical devices.

As shown in FIG. 1, system 100 includes IMD 110, leads 130A and 130B, and external programmer 150, shown in conjunction with a patient 105, who is ordinarily a human patient. In the example of FIG. 1, IMD 110 is an implantable electrical stimulator that is configured to generate and deliver electrical stimulation therapy to patient 105 via one or more electrodes of leads 130A and/or 130B (collectively, "leads 130"), e.g., for relief of chronic pain or other symptoms. In other examples, IMD 110 may be coupled to a single lead carrying multiple electrodes or more than two leads each carrying multiple electrodes. In some examples, the stimulation signals, or pulses, may be configured to elicit detectable ECAP signals that IMD 110 may use as feedback for adjusting stimulation parameter values, such as amplitude or electrode combination that defines subsequent stimulation pulses. IMD 110 may be a chronic electrical stimulator that remains implanted within patient 105 for weeks, months, or even years. In other examples, IMD 110 may be a temporary, or trial, stimulator used to screen or evaluate the efficacy of electrical stimulation for chronic therapy. In one example, IMD 110 is implanted within patient 105, while in another example, IMD 110 is an external device coupled to percutaneously implanted leads. In some examples, IMD 110 uses one or more leads, while in other examples, IMD 110 is leadless.

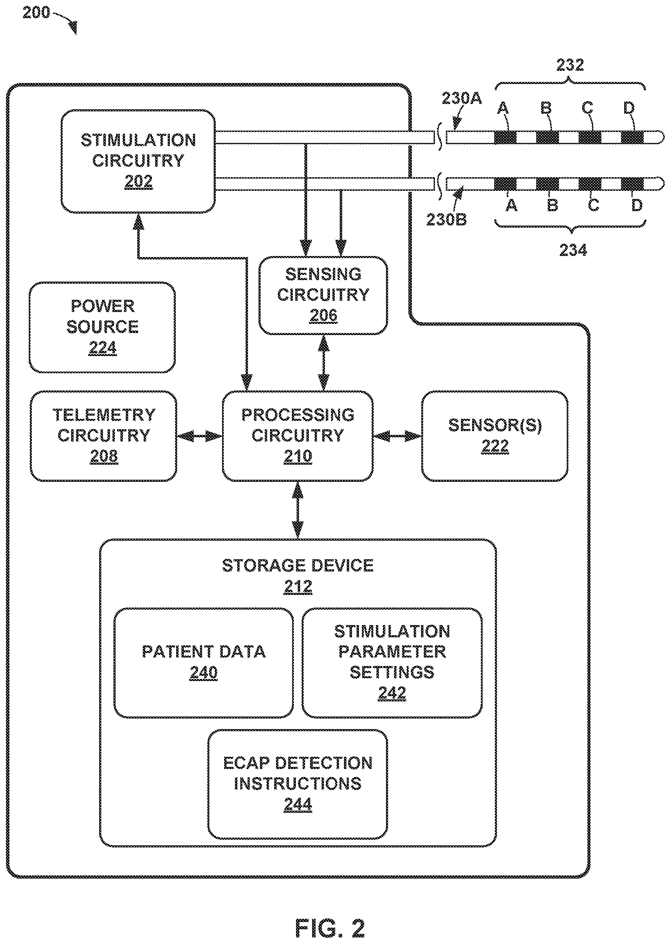

IMD 110 may be constructed of any polymer, metal, or composite material sufficient to house the components of IMD 110 (e.g., components illustrated in FIG. 2) within patient 105. In this example, IMD 110 may be constructed with a biocompatible housing, such as titanium or stainless steel, or a polymeric material such as silicone, polyurethane, or a liquid crystal polymer, and surgically implanted at a site in patient 105 near the pelvis, abdomen, or buttocks. In other examples, IMD 110 may be implanted within other suitable sites within patient 105, which may depend, for example, on the target site within patient 105 for the delivery of electrical stimulation therapy. The outer housing of IMD 110 may be configured to provide a hermetic seal for components, such as a rechargeable or non-rechargeable power source. Additionally or alternatively, the outer housing of IMD 110 may be selected from a material that facilitates receiving energy to charge the rechargeable power source.

Electrical stimulation energy, which may be constant-current or constant-voltage-based pulses, for example, is delivered from IMD 110 to one or more target tissue sites of patient 105 via one or more electrodes (not shown) of implantable leads 130. In the example of FIG. 1, leads 130 carry electrodes that are placed adjacent to the target tissue of spinal cord 120. One or more of the electrodes may be disposed at a distal tip of a lead 130 and/or at other positions at intermediate points along the lead. Leads 130 may be implanted and coupled to IMD 110. The electrodes may transfer electrical stimulation generated by an electrical stimulation generator of IMD 110 to tissue of patient 105. Although leads 130 may each be a single lead, lead 130 may include a lead extension or other segments that may aid in implantation or positioning of lead 130. In some other examples, IMD 110 may be a leadless stimulator with one or more arrays of electrodes arranged on a housing of the stimulator rather than leads that extend from the housing. In some other examples, system 100 may include one lead or more than two leads, each coupled to IMD 110 and directed to similar or different target tissue sites.

The electrodes of leads 130 may be electrode pads on a paddle lead, circular (e.g., ring) electrodes surrounding the body of the lead, conformable electrodes, cuff electrodes, segmented electrodes (e.g., electrodes disposed at different circumferential positions around the lead instead of a continuous ring electrode), any combination thereof (e.g., ring electrodes and segmented electrodes), or any other type of electrodes capable of forming unipolar, bipolar, or multipolar electrode combinations for therapy. Ring electrodes arranged at different axial positions at the distal ends of lead 130 will be described for purposes of illustration.

The deployment of electrodes via leads 130 is described for purposes of illustration, but arrays of electrodes may be deployed in different ways. For example, a housing associated with a leadless stimulator may carry arrays of electrodes, e.g., rows and/or columns (or other patterns), to which shifting operations may be applied. Such electrodes may be arranged as surface electrodes, ring electrodes, or protrusions. As a further alternative, electrode arrays may be formed by rows and/or columns of electrodes on one or more paddle leads. In some examples, electrode arrays include electrode segments, which are arranged at respective positions around a periphery of a lead, e.g., arranged in the form of one or more segmented rings around a circumference of a cylindrical lead. In other examples, one or more of leads 130 are linear leads having eight ring electrodes along the axial length of the lead. In another example, the electrodes are segmented rings arranged in a linear fashion along the axial length of the lead and at the periphery of the lead.

The stimulation parameter set of a therapy stimulation program, which defines the stimulation pulses of electrical stimulation therapy by IMD 110 through the electrodes of leads 130, may include information identifying which electrodes have been selected (e.g., electrode combination) for delivery of stimulation according to a stimulation program, the polarities of the selected electrodes, voltage or current amplitude, pulse frequency, pulse width, and/or a pulse shape of stimulation delivered by the electrodes. These stimulation parameter values may be predetermined parameter values defined by a user and/or automatically determined by system 100 based on one or more factors or user input.

Although FIG. 1 is directed to SCS therapy, e.g., stimulation delivered to the spinal cord and configured to treat pain, in other examples system 100 may be configured to treat any other condition that may benefit from electrical stimulation therapy. For example, system 100 may be used to treat tremor, Parkinson's disease, epilepsy, a pelvic floor disorder (e.g., urinary incontinence or other bladder dysfunction, fecal incontinence, pelvic pain, bowel dysfunction, or sexual dysfunction), obesity, gastroparesis, or psychiatric disorders (e.g., depression, mania, obsessive compulsive disorder, anxiety disorders, and the like). In this manner, system 100 may be configured to provide therapy taking the form of deep brain stimulation (DBS), peripheral nerve stimulation (PNS), peripheral nerve field stimulation (PNFS), cortical stimulation (CS), pelvic floor stimulation, gastrointestinal stimulation, or any other stimulation therapy capable of treating a condition of patient 105.

In some examples, lead 130 includes one or more sensors configured to allow IMD 110 to monitor one or more parameters of patient 105, such as patient activity, pressure, temperature, or other characteristics. The one or more sensors may be provided in addition to, or in place of, therapy delivery by lead 130.

IMD 110 is configured to deliver electrical stimulation therapy to patient 105 via selected combinations of electrodes carried by one or both of leads 130, alone or in combination with an electrode carried by or defined by an outer housing of IMD 110. The target tissue for the electrical stimulation therapy may be any tissue affected by electrical stimulation, which may be in the form of electrical stimulation pulses or continuous waveforms. In some examples, the target tissue includes nerves, smooth muscle, or skeletal muscle. In the example illustrated by FIG. 1, the target tissue is tissue proximate spinal cord 120, such as within an intrathecal space or epidural space of spinal cord 120, or, in some examples, adjacent nerves that branch off spinal cord 120. Leads 130 may be introduced into spinal cord 120 in via any suitable region, such as the thoracic, cervical, or lumbar regions. Stimulation of spinal cord 120 may, for example, prevent pain signals from traveling through spinal cord 120 and to the brain of patient 105. Patient 105 may perceive the interruption of pain signals as a reduction in pain and, therefore, efficacious therapy results. In other examples, stimulation of spinal cord 120 may produce paresthesia, which may reduce the perception of pain by patient 105, and thus, provide efficacious therapy results.

IMD 110 is configured to generate and deliver electrical stimulation therapy to a target stimulation site within patient 105 via the electrodes of leads 130 according to one or more therapy stimulation programs. A therapy stimulation program defines values for one or more parameters (e.g., a parameter set) that define an aspect of the therapy delivered by IMD 110. For example, a therapy stimulation program that controls delivery of stimulation by IMD 110 in the form of pulses may define values for voltage or current pulse amplitude, pulse width, pulse rate (e.g., pulse frequency), electrode combination (which may also specify electrode polarity), pulse shape, etc., for stimulation pulses delivered by IMD 110.

Furthermore, IMD 110 may be configured to deliver stimulation to patient 105 via a combination of electrodes of leads 130, alone or in combination with an electrode carried by or defined by an outer housing of IMD 110, in order to detect ECAP signals. The tissue targeted by the stimulation may be the same or similar tissue targeted by the electrical stimulation therapy, but IMD 110 may deliver stimulation pulses for ECAP signal detection via the same, at least some of the same, or different electrodes.

IMD 110 can deliver stimulation to a target stimulation site within patient 105 via the electrodes of leads 130 according to one or more ECAP stimulation programs to develop a growth curve of the ECAP. The one or more ECAP stimulation programs may be stored in a storage device of IMD 110 and/or external programmer 150. Each ECAP stimulation program of the one or more ECAP stimulation programs includes values for one or more parameters that define an aspect of the stimulation delivered by IMD 110 according to that program, such as current or voltage amplitude, pulse width, pulse frequency, electrode combination, etc. In some examples, the ECAP stimulation program may also define the number of pules and parameter values for each pulse of multiple pulses within a pulse "sweep" configured to obtain a plurality of ECAP signals for respective pulses of different parameter values (e.g., increasing or decreasing amplitudes) in order to obtain the growth curve that IMD 110 may use to determine an ECAP threshold (e.g., a neural threshold of the patient). In some examples, IMD 110 delivers stimulation to patient 105 according to multiple ECAP stimulation programs. Although these functions are described with respect to IMD 110, other devices, such as external programmer 150, may perform these functions, such as determining the estimated neural threshold based on the growth curve of ECAP characteristic values.

A user, such as a clinician or patient 105, may interact with a user interface of an external programmer 150 to program IMD 110. Programming of IMD 110 may refer generally to the generation and transfer of commands, programs, or other information to control the operation of IMD 110. In this manner, IMD 110 may receive the transferred commands and programs from external programmer 150 to control stimulation, such as electrical stimulation therapy to develop the growth curve. For example, external programmer 150 may transmit therapy stimulation programs, ECAP stimulation programs, stimulation parameter adjustments, therapy stimulation program selections, ECAP program selections, user input, or other information to control the operation of IMD 110, e.g., by wireless telemetry or wired connection.

In some cases, external programmer 150 may be characterized as a "physician programmer" or a "clinician programmer" if it is primarily intended for use by a physician or clinician. In other cases, external programmer 150 may be characterized as a "patient programmer" if it is primarily intended for use by a patient. A patient programmer may be generally accessible to patient 105 and, in many cases, may be a portable device that accompanies patient 105 throughout the patient's daily routine. For example, a patient programmer may receive input from patient 105 when the patient wishes to terminate or change electrical stimulation therapy, when a patient perceives stimulation being delivered or when a patient terminates therapy due to comfort level. In general, a physician or clinician programmer may support selection and generation of programs by a clinician for use by IMD 110, whereas a patient programmer may support adjustment and selection of such programs by a patient during ordinary use. In other examples, external programmer 150 may include, or be part of, an external charging device that recharges a power source of IMD 110. In this manner, a user may program and charge IMD 110 using one device, or multiple devices.

As described herein, information may be transmitted between external programmer 150 and IMD 110. For instance, IMD 110 and external programmer 150 may communicate via wireless communication using any techniques known in the art. Examples of communication techniques may include, for example, radiofrequency (RF) telemetry and inductive coupling, but other techniques are also contemplated. In some examples, external programmer 150 includes a communication head that may be placed proximate to the patient's body near the implant site of IMD 110 in order to improve the quality and/or security of communication between IMD 110 and external programmer 150. Communication between external programmer 150 and IMD 110 may occur during power transmission or separate from power transmission.

In some examples, IMD 110, in response to commands from external programmer 150, delivers electrical stimulation therapy according to a plurality of therapy stimulation programs to a target tissue site of the spinal cord 120 of patient 105 via electrodes (not depicted) on leads 130. In some examples, IMD 110 modifies therapy stimulation programs as therapy needs of patient 105 evolve over time. For example, the modification of the therapy stimulation programs may cause the adjustment of at least one parameter of the plurality of therapy pulses. When patient 105 receives the same therapy for an extended period, the efficacy of the therapy may be reduced. In some cases, parameters of the plurality of therapy pulses may be automatically updated. In some examples, IMD 110 may detect ECAP signals from pulses delivered for the purpose of providing therapy to the patient.

In some examples, efficacy of electrical stimulation therapy may be indicated by one or more characteristics of an action potential that is evoked by a stimulation pulse delivered by IMD 110, for example, by determining an ECAP characteristic value of the ECAP signal. Electrical stimulation therapy delivery by leads 130 of IMD 110 may cause neurons within the target tissue to evoke a compound action potential that travels up and down the target tissue, eventually arriving at sensing electrodes of IMD 110. Furthermore, stimulation pulses may also elicit at least one ECAP signal, and ECAPs responsive to stimulation may also be a surrogate for the effectiveness of the therapy and/or the intensity perceived by the patient. The amount of action potentials (e.g., number of neurons propagating action potential signals) that are evoked may be based on the various parameters of electrical stimulation pulses such as amplitude, pulse width, frequency, pulse shape (e.g., slew rate at the beginning and/or end of the pulse), etc. The slew rate may define the rate of change of the voltage and/or current amplitude of the pulse at the beginning and/or end of each pulse or each phase within the pulse. For example, a very high slew rate indicates a steep or even near-vertical edge of the pulse, and a low slew rate indicates a longer ramp up (or ramp down) in the amplitude of the pulse. In some examples, these parameters contribute to an intensity of the electrical stimulation. In addition, a characteristic of the ECAP signal (e.g., an amplitude) may change based on the distance between the stimulation electrodes and the nerves subject to the electrical field produced by the delivered control stimulation pulses.

Example techniques for adjusting stimulation parameter values for pulses (e.g., pulses configured to contribute to therapy for the patient) are based on comparing the value of a characteristic of a measured ECAP signal to a target ECAP characteristic value. In some examples, the target ECAP characteristic value may be an ECAP threshold (e.g., a neural threshold) or a value calculated based on the neural threshold (e.g., a percentage below or above 100% of the neural threshold). During delivery of control stimulation pulses defined by one or more ECAP test stimulation programs, IMD 110, via two or more electrodes interposed on leads 130, senses electrical potentials of tissue of the spinal cord 120 of patient 105 to measure the electrical activity of the tissue. IMD 110 senses ECAPs from the target tissue of patient 105, e.g., with electrodes on one or more leads 130 and associated sensing circuitry. In some examples, IMD 110 receives a signal indicative of the ECAP from one or more sensors, e.g., one or more electrodes and circuitry, internal or external to patient 105. Such a signal may indicate an ECAP of the tissue of patient 105.

In the examples described above, IMD 110 is described as performing a plurality of processing and computing functions. However, external programmer 150 instead may perform one, several, or all of these functions. In this alternative example, IMD 110 relays sensed signals to external programmer 150 for analysis, and external programmer 150 transmits instructions to IMD 110 to adjust the one or more parameters defining the electrical stimulation therapy based on analysis of the sensed signals. For example, IMD 110 may relay the sensed signal indicative of an ECAP to external programmer 150. External programmer 150 may compare the parameter value of the ECAP to the target ECAP characteristic value, and in response to the comparison, external programmer 150 may instruct IMD 110 to adjust one or more stimulation parameters that define subsequent electrical stimulation pulses delivered to patient 105.

In some examples, the stimulation parameters and the target ECAP characteristic values associated with the ECAP threshold may initially be set at the clinic, but may be subsequently set and/or adjusted at home by patient 105. For example, the target ECAP characteristics may be changed to match, or to be a fraction of, or a multiplier of, an ECAP threshold. In some examples, target ECAP characteristics may be specific to respective different posture states of the patient (which the system may detect via the ECAP signal and/or a posture sensor which may include an accelerometer). Once the target ECAP characteristic values are set, the example techniques allow for automatic adjustment of parameter values that define stimulation pulses to maintain a consistent volume of neural activation and consistent perception of therapy for the patient. The ability to change the stimulation parameter values may also allow the therapy to have long-term efficacy, with the ability to keep the intensity of the stimulation (e.g., as indicated by the ECAP) consistent by comparing the measured ECAP values to the target ECAP characteristic value. In addition, or alternatively, to maintaining stimulation intensity, IMD 110 may monitor the characteristic values of the ECAP signals to limit one or more parameter values that define stimulation pulses. IMD 110 may perform these changes without intervention by a physician or patient 105.

In some examples, system 100 changes the target ECAP characteristic value over a period of time, such as according to a change to an ECAP threshold (e.g., a perception threshold or detection threshold). The system may be programmed to change the target ECAP characteristic in order to adjust the intensity of stimulation pulses to provide varying sensations to the patient (e.g., increase or decrease the volume of neural activation). Although the system may change the target ECAP characteristic value, received ECAP signals may still be used by the system to adjust one or more parameter values of the stimulation pulse in order to meet the target ECAP characteristic value.

One or more devices within system 100, such as IMD 110 and/or external programmer 150, may perform various functions as described herein. For example, IMD 110 may include stimulation circuitry configured to deliver electrical stimulation, sensing circuitry configured to sense a plurality ECAP signals, and processing circuitry. The processing circuitry may be configured to control the stimulation circuitry to deliver a plurality of electrical stimulation pulses having different amplitude values and control the sensing circuitry to detect, after delivery of each electrical stimulation pulse, a respective ECAP signal, and to determine ECAP characteristic values for each of the ECAP signals. The processing circuitry of IMD 110 may then determine, based on the plurality of ECAP characteristic values, an ECAP threshold (e.g., a neural threshold) of a patient. The neural threshold may be similar to a perception threshold that the patient would have manually identified during the sweep of pulses defined by increasing amplitude values within the sweep. As such, IMD 110, or another device such as external programmer 150, may automatically determine the neural threshold, e.g., without patient input.

In some examples, IMD 110 may include the stimulation circuitry, the sensing circuitry, and the processing circuitry. However, in other examples, one or more additional devices may be part of the system that performs the functions described herein. For example, IMD 110 may include the stimulation circuitry and the sensing circuitry, but external programmer 150 or another external device may include the processing circuitry that at least determines the neural threshold of the patient. IMD 110 may transmit the sensed ECAP signals, or data representing the ECAP signal, to external programmer 150, for example. Therefore, the processes described herein may be performed by multiple devices in a distributed system. In some examples, system 100 may include one or more electrodes that deliver and/or sense electrical signals. Such electrodes may be configured to sense the ECAP signals. In some examples, the same electrodes may be configured to sense signals representative of transient movements of the patient. In other examples, other sensors, such as accelerometers, gyroscopes, or other movement sensors may be configured to sense movement of the patient that indicates that the patient may have transitioned to a different posture state.

As described herein, the processing circuitry of IMD 110 may be configured to determine characteristic values for each of the plurality of ECAP signals detected after each of the plurality of electrical stimulation pulses. A plurality of stimulation pulses is delivered, where each stimulation pulse may be defined by a different respective value of a stimulation parameter. The plurality of stimulation pulses may include increasing amplitudes to elicit different responses of ECAP signal information. In one or more examples, the characteristic value for each ECAP signal is a representation of the ECAP signal according to some metric, and is determined by IMD 110, for example, by removing an artifact from the ECAP signal. These characteristic values may thus be used as a metric derived from the ECAP signal that represents the relative nerve fiber activation caused by the delivered stimulation pulse. In this manner, each ECAP signal is associated with a respective characteristic value of the characteristic values. As long as the distance between the electrodes and target nerve remains relatively constant during delivery of the pulses and sensing of the respective ECAP signals, higher amplitude pulses generally cause more neural recruitment and larger ECAP signals.

The processing circuitry of IMD 110 may be configured to determine an ECAP threshold, such as a neural threshold, of a patient based on characteristic values for the plurality of ECAP signals detected after each of the plurality of electrical stimulation pulses. For example, the neural threshold may be determined by sweeping through a plurality of amplitudes for respective stimulation pulses and generating a growth curve from the sensed ECAP signals. The growth curve, or a portion of the growth curve, may be used by the system to determine a neural threshold, for example, when the growth curve transitions from a first linear region to a second curvilinear region. In some examples, the system may determine a therapeutic range based on one or more characteristics of the second curvilinear region (e.g., radius of curvature, width of current amplitude of the curvilinear region, ratio of ECAP amplitude width and current amplitude width of the curvilinear region, etc.). In this manner, as further described herein, the system may use this sweep of pulses, or as part of additional sweeps of pulses varying one or more parameter values, to automatically determine parameter values for therapy based on ECAP characteristic(s).

The sweep of amplitudes for stimulation pulses may be linear, non-linear, or even adaptive based on sensed information. In one or more examples, the IMD (or external programmer) may step through the first linear region by increasing a value of one or more of the plurality of stimulation parameters in greater steps (e.g., a faster rate of change), and once an inflection in the curve is sensed, the system may reduce the rate of change for stimulation amplitude to slow the stepping of changes (i.e., adaptive stepping). In one or more examples, the processing circuitry may increase the value of the stimulation parameter until the neural threshold is determined or can be determined from the data obtained so far in the sweep, and then stop delivering stimulation for the remaining sweep because it is no longer needed. In other examples, the system may continue performing the sweep of amplitudes until a predetermined amplitude value is reached or the system receives input from the patient requesting that stimulation be stopped (e.g., the patient has reached a discomfort threshold). If input from the patient indicates that the discomfort threshold has been reached, the system may set the discomfort threshold stimulation amplitude as the upper threshold for stimulation during therapy.

In some examples, the system may utilize certain constraints on the process for determining the ECAP threshold from a sweep of pulses defined by different stimulation parameter values. For example, processing circuitry may be configured to receive a maximum amplitude value for a plurality of stimulation pulses. The maximum amplitude value may be set by user input received via a user interface. For example, a clinician may know that a patient may prefer not to receive stimulation pulses over a certain amplitude or that generally patients prefer to have stimulation amplitudes below the maximum amplitude value.

The processing circuitry can then control stimulation generation circuitry to begin delivery of the plurality of stimulation pulses at an initial amplitude value less than the maximum amplitude value. In some examples, the initial amplitude value may be zero, but in other examples, the initial amplitude value may be greater than zero and a value less than typically elicits an ECAP signal. The processing circuitry can then iteratively increase a subsequent amplitude value of a next stimulation pulse of the plurality of stimulation pulses up to the maximum amplitude value. This process may be referred to as the sweep of pulses. The processing circuitry may be configured to receive, for each stimulation pulse of the plurality of stimulation pulses, ECAP signal information, wherein the ECAP signal information comprises ECAP signals sensed by sensing circuitry. The processing circuitry can then determine, from the ECAP signal information for each stimulation pulse, ECAP characteristic values for each of the ECAP signals and determine, based on the ECAP characteristic values and during the delivery of the stimulation pulses with iteratively increasing amplitude values, at least one threshold from which one or more stimulation parameter values for subsequent stimulation pulses are determined. This threshold may be referred to as an ECAP threshold, and may include thresholds such as a neural threshold for the electrode combination that delivered the stimulation pulses that were part of the sweep. The processing circuitry may analyze the ECAP characteristic values received after each delivered stimulation pulse to determine if the already received ECAP characteristic values indicate the ECAP threshold. Therefore, the processing circuitry can terminate delivery of the stimulation pulses as soon as the processing circuitry can determine the ECAP threshold. In other examples, the plurality of stimulation pulses begin at an initial amplitude value less than the maximum amplitude value, and the processing circuitry is configured to iteratively increase a subsequent amplitude value of a next stimulation pulse of the plurality of stimulation pulses up to the maximum amplitude value. In some examples, the ECAP characteristic values are indicative of a direct measurement between an N1 peak and a P2 peak of the ECAP signal information for each ECAP signal of the ECAP signals.

The system may also present information associated with the maximum amplitude value for the sweep of pulses. For example, the processing circuitry is configured to receive the maximum amplitude value via a user interface and control the user interface to display a representation of a range of amplitude values for the plurality of stimulation pulses deliverable during the calibration routine of the sweep of pulses. The processing circuitry may then receive, via the user interface, user input specifying the maximum amplitude value and control user interface to display a representation of the at least one threshold. In some examples, the processing circuitry is configured to present the at least one threshold on the representation of the range of amplitude values. In some examples, the processing circuitry is configured to generate a graph of the ECAP characteristic values for each of the subsequent amplitude values and control a user interface to display updates to the graph as additional ECAP characteristic values are determined from delivered stimulation pulses of the plurality of stimulation pulses of the sweep of pulses. In some examples, the processing circuitry can control the user interface to display multiple graphs of ECAP characteristic values for each electrode combination used to deliver stimulation pulses for the respective sweeps of pulses being interleaved.

In some examples, the processing circuitry is configured to control the stimulation generation circuitry to interleave the delivery the plurality of stimulation pulses from a first electrode combination and additional stimulation pulses from a second electrode combination, and then receive, for each stimulation pulse of the plurality of stimulation pulses and the additional stimulation pulses, ECAP signal information. The processing circuitry may also be configured to determine, from the ECAP signal information for each stimulation pulse, ECAP characteristic values for each of the ECAP signals and control a user interface to display a graph of the ECAP characteristic values for each of the subsequent amplitude values for each of the plurality of stimulation pulses from the first electrode combination and the additional stimulation pulses from the second electrode combination. Based on the ECAP characteristic values and during the delivery of the stimulation pulses and additional stimulation pulses with iteratively increasing amplitude values, the processing circuitry may determine at least one threshold from which one or more stimulation parameter values for subsequent stimulation pulses are determined for each of the first electrode combination and the second electrode combination.

As described herein, the ECAP threshold determined as a result of the sweep of pulses may be a neural threshold. In some examples, this neural threshold is a perception threshold for the patient. The ECAP characteristic values from which the ECAP threshold is determined may create a growth curve of ECAP characteristic values for different amplitudes or other parameter value. In some examples, the processing circuitry is configured to determine the at least one threshold at least in part on a curvature of an inflection region of a growth curve corresponding to the ECAP characteristic values. The curvature may be a graph based determination or determined based on deviation of subsequent ECAP characteristic values from a rolling average or other metric representative of the ECAP characteristic values already received as part of the sweep of pulses. In some examples, the processing circuitry is configured to terminate the delivery of the plurality of stimulation pulses in response to determining the at least one threshold. In other words, even if there are more pulses of the sweep remaining to be delivered, the processing circuitry may truncate the sweep and prevent further pulses of the sweep being delivered.

The processing circuitry functionality described herein may be located on one or more devices of a system, such as system 100. In one example, IMD 110 includes at least a portion of the processing circuitry. In one example, external programmer 150 includes at least a portion of the processing circuitry and a user interface configured to receive user input identifying the maximum amplitude value. In this manner, the functionality described herein may be contained within a single device or distributed over two or more devices of the system.

In one example, system 100 (which may be or may include IMD 110, external programmer 150, and/or off-site or networked computing systems) may include a stimulation generator configured to deliver a stimulation pulse to patient 105, and sensing circuitry configured to sense an ECAP signal evoked by the stimulation pulse. System 100 may also include processing circuitry configured to determine ECAP characteristic values for each of the ECAP signals, and determine a targeted range of ECAP characteristic values based on the growth curve that is based on the ECAP threshold, which may be a range, a characteristic value of the targeted ECAP signal, and at least one parameter value at least partially defining electrical stimulation therapy to be delivered or offered to the patient. The patient or clinician may further modify the stimulation therapy, for example, based on patient preference or expected battery life, for example.

In one example, IMD 110 may determine a target ECAP characteristic value based on the ECAP threshold, such as a neural response, and calculate at least one parameter value according to a difference between the current ECAP characteristic value. In this manner, IMD 110 may deliver stimulation in closed-loop fashion using ECAP characteristic values as feedback. Processing circuitry of IMD 110 may thus be configured to control the stimulation generator to deliver the electrical stimulation therapy to the patient according to at least one adjusted parameter value, which may be selected based on the ECAP characteristic values and/or ECAP threshold. IMD 110 may include stimulation circuitry, sensing circuitry, and processing circuitry. In some examples, other devices, such as an external device or different implanted device, may analyze ECAP signals for characteristic values and/or adjust parameter values that define stimulation pulses based on the characteristic values.

Although in the example of FIG. 1, IMD 110 takes the form of an SCS device, in other examples, IMD 110 takes the form of any combination of deep brain stimulation (DBS) devices, peripheral nerve stimulators, implantable cardioverter defibrillators (ICDs), pacemakers, cardiac resynchronization therapy devices (CRT-Ds), left ventricular assist devices (LVADs), implantable sensors, orthopedic devices, or drug pumps, as examples. Moreover, techniques of this disclosure may be used to determine stimulation thresholds (e.g., perception thresholds and detection thresholds) associated any one of the aforementioned IMDs and then use a stimulation threshold to inform the intensity (e.g., stimulation levels) of therapy.

FIG. 2 is a block diagram illustrating an example configuration of components of an IMD 200. IMD 200 may be an example of IMD 110 of FIG. 1. In the example shown in FIG. 2, IMD 200 includes stimulation generation circuitry 202, sensing circuitry 206, telemetry circuitry 208, processing circuitry 210, storage device 212, sensor(s) 222, and power source 224.

In the example shown in FIG. 2, storage device 212 stores patient data 240, stimulation parameter settings 242, and ECAP detection instructions 244 in separate memories within storage device 212 or separate areas within storage device 212. Patient data 240 may include parameter values, target characteristic values, or other information specific to the patient. In some examples, stimulation parameter settings 242 may include stimulation parameter values for respective different stimulation programs selectable by the clinician or patient for therapy. In this manner, each stored therapy stimulation program, or set of stimulation parameter values, of stimulation parameter settings 242 defines values for a set of electrical stimulation parameters (e.g., a stimulation parameter set), such as a stimulation electrode combination, electrode polarity, current or voltage amplitude, pulse width, pulse rate, pulse shape, and/or duty cycle. Storage device 212 may also store ECAP detection instructions 244 that define values for a set of electrical stimulation parameters configured to elicit a detectable ECAP signal, such as a stimulation electrode combination, electrode polarity, current or voltage amplitude, pulse width, pulse rate, and/or pulse shape. ECAP detection instructions 244 may also have additional information such as instructions regarding when to deliver control pulses based on the pulse width and/or frequency of the pulses defined in stimulation parameter settings 242, detection windows for detecting ECAP signals, instructions for determining characteristic values from ECAP signals, etc. For example, ECAP detection instructions 244 may define how characteristic values of ECAP signals are to be determined. ECAP detection instructions 244 may also, in some examples, include instructions for performing sweeps of pulses for obtaining growth curves and instructions for determining ECAP thresholds from the ECAP characteristic values obtained from the sweeps of pulses.

Accordingly, in some examples, stimulation generation circuitry 202 generates electrical stimulation signals in accordance with the electrical stimulation parameters noted above. Other ranges of stimulation parameter values may also be useful and may depend on the target stimulation site within patient 105. While stimulation "pulses" are primarily described herein, stimulation signals may be of any form, such as continuous-time signals (e.g., sine waves) or the like. Stimulation generation circuitry 204 may include independently controllable current sinks and sources for respective electrodes 232, 234. For example, stimulation generation circuitry 204 comprises a plurality of pairs of voltage sources, current sources, voltage sinks, or current sinks connected to each of electrodes 232, 234 such that each pair of electrodes has a unique signal circuit. In other words, in these examples, each of electrodes 232, 234 is independently controlled via its own signal circuit (e.g., via a combination of a regulated voltage source and sink or regulated current source and sink), as opposed to switching signals between electrodes 232, 234. In this manner, processing circuitry 208 may control switches or transistors to selective couple the sources and/or sinks to the conductor of electrodes of an electrode combination.

One or more switches (not shown) may selectively couple sensing circuitry 206 to respective electrodes in order to sense signals via two or more electrodes 232, 234. In other examples, switch circuitry may include one or more switch arrays, one or more multiplexers, one or more switches (e.g., a switch matrix or other collection of switches), or other electrical circuitry configured to direct stimulation signals from stimulation generation circuitry 204 to one or more of electrodes 232, 234, or directed sensed signals from one or more of electrodes 232, 234 to sensing circuitry 206. In other examples, stimulation generation circuitry 204 and/or sensing circuitry 206 may include sensing circuitry to direct signals to and/or from one or more of electrodes 232, 234, which may or may not also include switch circuitry.

Sensing circuitry 206 is configured to monitor signals from any combination of electrodes 232, 234. In some examples, sensing circuitry 206 includes one or more amplifiers, filters, and/or analog-to-digital converters. Sensing circuitry 206 may be used to sense physiological signals, such as ECAP signals. In some examples, sensing circuitry 206 detects ECAPs from a particular combination of electrodes 232, 234. In some cases, the particular combination of electrodes for sensing ECAPs includes different electrodes than a set of electrodes 232, 234 used to deliver stimulation pulses. Alternatively, in other cases, the particular combination of electrodes used for sensing ECAPs includes at least one of the same electrodes as a set of electrodes used to deliver stimulation pulses to patient 105. Sensing circuitry 206 may provide signals to an analog-to-digital converter for conversion into a digital signal for processing, analysis, storage, and/or output by processing circuitry 210.

Telemetry circuitry 208 supports wireless communication between IMD 200 and an external programmer (not shown in FIG. 2) or another computing device under the control of processing circuitry 210. Processing circuitry 210 of IMD 200 may receive, as updates to programs, values for various stimulation parameters (e.g., amplitude and electrode combination) from the external programmer via telemetry circuitry 208. Processing circuitry 210 may store updates to the stimulation parameter settings 242 or any other data in storage device 212. Telemetry circuitry 208 in IMD 200, as well as telemetry circuits in other devices and systems described herein, such as the external programmer, may accomplish communication by radiofrequency (RF) communication techniques. In addition, telemetry circuitry 208 may communicate with an external medical device programmer (not shown in FIG. 2) via proximal inductive interaction of IMD 200 with the external programmer. The external programmer may be one example of external programmer 150 of FIG. 1. Accordingly, telemetry circuitry 208 may send information to the external programmer on a continuous basis, at periodic intervals, or upon request from IMD 110 or the external programmer.

Processing circuitry 210 may include any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 210 herein may be embodied as firmware, hardware, software or any combination thereof. Processing circuitry 210 controls stimulation generation circuitry 202 to generate stimulation signals according to stimulation parameter settings 242 and any other instructions stored in storage device 212 to apply stimulation parameter values specified by one or more of programs, such as amplitude, pulse width, pulse rate, and pulse shape of each of the stimulation signals.

In the example shown in FIG. 2, the set of electrodes 232 includes electrodes 232A, 232B, 232C, and 232D, and the set of electrodes 234 includes electrodes 234A, 234B, 234C, and 234D. In other examples, a single lead may include all eight electrodes 232 and 234 along a single axial length of the lead. Processing circuitry 210 also controls stimulation generation circuitry 202 to generate and apply the stimulation signals to selected combinations of electrodes 232, 234. In some examples, stimulation generation circuitry 202 includes a switch circuit that may couple stimulation signals to selected conductors within leads 230, which, in turn, deliver the stimulation signals across selected electrodes 232, 234. Such a switch circuit may be a switch array, switch matrix, multiplexer, or any other type of switching circuit configured to selectively couple stimulation energy to selected electrodes 232, 234 and to selectively sense bioelectrical neural signals of a spinal cord of the patient (not shown in FIG. 2) with selected electrodes 232, 234.

In other examples, however, stimulation generation circuitry 202 does not include a switch circuit and switch circuitry 204 does not interface between stimulation generation circuitry 202 and electrodes 232, 234. In these examples, stimulation generation circuitry 202 includes a plurality of pairs of voltage sources, current sources, voltage sinks, or current sinks connected to each of electrodes 232, 234 such that each pair of electrodes has a unique signal circuit. In other words, in these examples, each of electrodes 232, 234 is independently controlled via its own signal circuit (e.g., via a combination of a regulated voltage source and sink or regulated current source and sink), as opposed to switching signals between electrodes 232, 234.

Electrodes 232, 234 on respective leads 230 may be constructed of a variety of different designs. For example, one or both of leads 230 may include one or more electrodes at each longitudinal location along the length of the lead, such as one electrode at different perimeter locations around the perimeter of the lead at each of the locations A, B, C, and D. In one example, the electrodes may be electrically coupled to stimulation generation circuitry 202, e.g., switching circuitry of the stimulation generation circuitry 202, via respective wires that are straight or coiled within the housing of the lead and run to a connector at the proximal end of the lead. In another example, each of the electrodes of the lead may be electrodes deposited on a thin film. The thin film may include an electrically conductive trace for each electrode that runs the length of the thin film to a proximal end connector. The thin film may then be wrapped (e.g., a helical wrap) around an internal member to form the lead 230. These and other constructions may be used to create a lead with a complex electrode geometry.

Although sensing circuitry 206 is incorporated into a common housing with stimulation generation circuitry 202 and processing circuitry 210 in FIG. 2, in other examples, sensing circuitry 206 may be in a separate housing from IMD 200 and may communicate with processing circuitry 210 via wired or wireless communication techniques. In some examples, one or more of electrodes 232 and 234 are suitable for sensing the ECAPs. For instance, electrodes 232 and 234 may sense the voltage amplitude of a portion of the ECAP signals, where the sensed voltage amplitude, such as the voltage difference between features within the signal, is a characteristic the ECAP signal.

Storage device 212 may be configured to store information within IMD 200 during operation. Storage device 212 may include a computer-readable storage medium or computer-readable storage device. In some examples, storage device 212 includes one or more of a short-term memory or a long-term memory. Storage device 212 may include, for example, random access memories (RAM), dynamic random access memories (DRAM), static random access memories (SRAM), magnetic discs, optical discs, flash memories, or forms of electrically programmable memories (EPROM) or electrically erasable and programmable memories (EEPROM). In some examples, storage device 212 is used to store data indicative of instructions for execution by processing circuitry 210. As discussed above, storage device 212 is configured to store patient data 240, stimulation parameter settings 242, and ECAP detection instructions 244.

In some examples, storage device 212 may store instructions on how processing circuitry 210 can adjust stimulation pulses in response to the determined characteristic values of ECAP signals. For example, processing circuitry 210 may monitor ECAP characteristic values obtained from ECAP signals (or a signal derived from the ECAP signal) to modulate stimulation parameter values (e.g., increase or decrease stimulation intensity to maintain a target therapeutic effect). In some examples, a target ECAP characteristic value may vary for different situations for a patient, such as different posture states, times of day, activities, etc.

Sensor(s) 222 may include one or more sensing elements that sense values of a respective patient parameter, such as posture state. As described, electrodes 232 and 234 may be the electrodes that sense the characteristic value of the ECAP signal. Sensor(s) 222 may include one or more accelerometers, optical sensors, chemical sensors, temperature sensors, pressure sensors, or any other types of sensors. Sensor(s) 222 may output patient parameter values that may be used as feedback to control delivery of therapy. For example, sensor(s) 222 may indicate patient activity, and processing circuitry 210 may increase the frequency of control pulses and ECAP sensing in response to detecting increased patient activity. In one example, processing circuitry 210 may initiate control pulses and corresponding ECAP sensing in response to a signal from sensor(s) 222 indicating that patient activity has exceeded an activity threshold. Conversely, processing circuitry 210 may decrease the frequency of control pulses and ECAP sensing in response to detecting decreased patient activity. For example, in response to sensor(s) 222 no longer indicating that the sensed patient activity exceeds a threshold, processing circuitry 210 may suspend or stop delivery of control pulses and ECAP sensing. In this manner, processing circuitry 210 may dynamically deliver control pulses and sense ECAP signals based on patient activity to reduce power consumption of the system when the electrode-to-neuron distance is not likely to change, and may increase a system response to ECAP changes when electrode-to-neuron distance is likely to change. IMD 200 may include additional sensors within the housing of IMD 200 and/or coupled via one of leads 130 or other leads. In addition, IMD 200 may receive sensor signals wirelessly from remote sensors via telemetry circuitry 208, for example. In some examples, one or more of these remote sensors may be external to patient (e.g., carried on the external surface of the skin, attached to clothing, or otherwise positioned external to patient 105). In some examples, signals from sensor(s) 222 indicate a position or body state (e.g., sleeping, awake, sitting, standing, or the like), and processing circuitry 210 may select target ECAP characteristic values according to the indicated position or body state.

Power source 224 is configured to deliver operating power to the components of IMD 200. Power source 224 may include a battery and a power generation circuit to produce the operating power. In some examples, the battery is rechargeable to allow extended operation. In some examples, recharging is accomplished through proximal inductive interaction between an external charger and an inductive charging coil within IMD 200. Power source 224 may include any one or more of a plurality of different battery types, such as nickel cadmium batteries and lithium ion batteries.

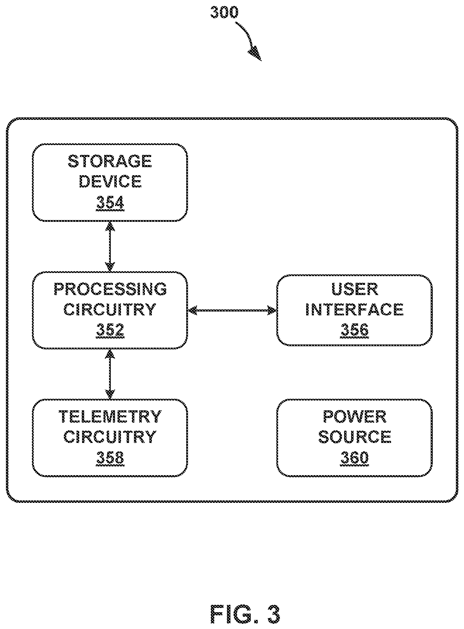

FIG. 3 is a block diagram illustrating an example configuration of components of an example external programmer 300. External programmer 300 may be an example of external programmer 150 of FIG. 1. Although external programmer 300 may generally be described as a handheld device, external programmer 300 may be a larger portable device or a more stationary device. In other examples, external programmer 300 may be included as part of an external charging device or may include the functionality of an external charging device. As illustrated in FIG. 3, external programmer 300 may include processing circuitry 352, storage device 354, user interface 356, telemetry circuitry 358, and power source 360.

Storage device 354 may store instructions that, when executed by processing circuitry 352, cause processing circuitry 352 and external programmer 300 to provide the functionality ascribed to external programmers 150, 300 throughout this disclosure. Each of these components, circuitry, or modules may include electrical circuitry that is configured to perform some, or all of the functionality described herein. For example, processing circuitry 352 may include processing circuitry configured to perform the processes discussed with respect to processing circuitry 352.

In general, external programmer 300 includes any suitable arrangement of hardware, alone or in combination with software and/or firmware, to perform the techniques attributed to external programmer 300, and processing circuitry 352, user interface 356, and telemetry circuitry 358 of external programmer 300. In various examples, external programmer 300 may include one or more processors, such as one or more microprocessors, DSPs, ASICs, FPGAs, or any other equivalent integrated or discrete logic circuitry, as well as any combinations of such components. External programmer 300 also, in various examples, may include a storage device 354, such as RAM, ROM, PROM, EPROM, EEPROM, flash memory, a hard disk, a CD-ROM, including executable instructions for causing the one or more processors to perform the actions attributed to them. Moreover, although processing circuitry 352 and telemetry circuitry 358 are described as separate modules, in some examples, processing circuitry 352 and telemetry circuitry 358 are functionally integrated. In some examples, processing circuitry 352 and telemetry circuitry 358 correspond to individual hardware units, such as ASICs, DSPs, FPGAs, or other hardware units.

Storage device 354 (e.g., a memory or other device configured to store data) may store instructions that, when executed by processing circuitry 352, cause processing circuitry 352 and external programmer 300 to provide the functionality ascribed to external programmers 150, 300 throughout this disclosure. For example, storage device 354 may include instructions that cause processing circuitry 352 to obtain a parameter set from memory, select a spatial electrode pattern, receive a user input and send a corresponding command to IMD 200, or any other functionality. Storage device 354 may include a plurality of programs, where each program includes a parameter set that defines therapy stimulation or control stimulation. Storage device 354 may also store data received from a medical device (e.g., IMD 110). For example, storage device 354 may store ECAP-related data recorded at a sensing module of the medical device, and storage device 354 may also store data from one or more sensors of the medical device.

User interface 356 may include a button or keypad, lights, a speaker for voice commands, a display, such as a liquid crystal display (LCD), light-emitting diode (LED), or organic light-emitting diode (OLED). In some examples the display includes a touchscreen. User interface 356 may be configured to display any information related to the delivery of electrical stimulation, identified posture states, sensed patient parameter values, or any other such information. User interface 356 may also receive user input (e.g., indication of when the patient perceives a stimulation pulse) via user interface 356. The input may be, for example, in the form of pressing a button on a keypad or selecting an icon from a touchscreen. The input may request starting or stopping electrical stimulation, a new spatial electrode pattern or a change to an existing spatial electrode pattern, or some other change to the delivery of electrical stimulation.

Telemetry circuitry 358 may support wireless communication between the medical device and external programmer 300 under the control of processing circuitry 352. Telemetry circuitry 358 may also be configured to communicate with another computing device via wireless communication techniques, or direct communication through a wired connection. In some examples, telemetry circuitry 358 provides wireless communication via an RF or proximal inductive medium. In some examples, telemetry circuitry 358 includes an antenna, which may take on a variety of forms, such as an internal or external antenna.

Examples of local wireless communication techniques that may be employed to facilitate communication between external programmer 300 and IMD 110 include RF communication according to the 802.11 or Bluetooth^{®} specification sets or other standard or proprietary telemetry protocols. In this manner, other external devices may be capable of communicating with external programmer 300 without needing to establish a secure wireless connection. As described herein, telemetry circuitry 358 may be configured to transmit a spatial electrode movement pattern or other stimulation parameter values to IMD 110 for delivery of electrical stimulation therapy. Although IMD 110 may determine characteristic values for ECAP signals and control the adjustment of stimulation parameter values in some examples, programmer 300 may perform these tasks alone or together with IMD 110 in a distributed function.

In some examples, selection of stimulation parameters or therapy stimulation programs are transmitted to the medical device for delivery to a patient (e.g., patient 105 of FIG. 1). In other examples, the therapy may include medication, activities, or other instructions that patient 105 must perform themself or a caregiver perform for patient 105. In some examples, external programmer 300 provides visual, audible, and/or tactile notifications that indicate there are new instructions. External programmer 300 requires receiving user input acknowledging that the instructions have been completed in some examples.

User interface 356 of external programmer 300 may also be configured to receive an indication from a clinician instructing a processor of the medical device to update one or more therapy stimulation programs or to update the target characteristic values for ECAP signals. Updating therapy stimulation programs and target characteristic values may include changing one or more parameters of the stimulation pulses delivered by the medical device according to the programs, such as amplitude, pulse width, frequency, and/or pulse shape of the therapy pulses and/or control pulses. User interface 356 may also receive instructions from the clinician commanding any electrical stimulation, including therapy stimulation and control stimulation, to commence or to cease. User interface 356 may also receive user input and/or display information as described herein.

Power source 360 is configured to deliver operating power to the components of external programmer 300. Power source 360 may include a battery and a power generation circuit to produce the operating power. In some examples, the battery is rechargeable to allow extended operation. Recharging may be accomplished by electrically coupling power source 360 to a cradle or plug that is connected to an alternating current (AC) outlet. In addition, recharging may be accomplished through proximal inductive interaction between an external charger and an inductive charging coil within external programmer 300. In other examples, traditional batteries (e.g., nickel cadmium or lithium ion batteries) may be used. In addition, external programmer 300 may be directly coupled to an alternating current outlet to operate.

The architecture of external programmer 300 illustrated in FIG. 3 is shown as an example. The techniques as set forth in this disclosure may be implemented in the example external programmer 300 of FIG. 3, as well as other types of systems not described specifically herein. Nothing in this disclosure should be construed so as to limit the techniques of this disclosure to the example architecture illustrated by FIG. 3.

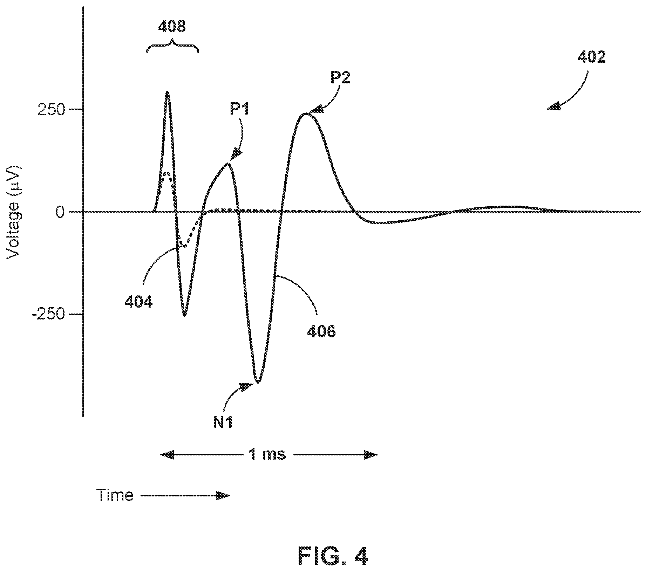

FIG. 4 is a graph 402 of example evoked compound action potentials (ECAPs) sensed for respective stimulation pulses, in accordance with one or more techniques of this disclosure. As shown in FIG. 4, graph 402 shows example ECAP signal 404 (dotted line) and ECAP signal 406 (solid line). In some examples, each of ECAP signals 404 and 406 are sensed from stimulation pulses that were delivered from a guarded cathode, where the control pulses are biphasic pulses including an interphase interval between each positive and negative phase of the pulse. In some such examples, the guarded cathode includes stimulation electrodes located at the end of an 8-electrode lead (e.g., leads 130 of FIG. 1) while two sensing electrodes are provided at the other end of the 8-electrode lead. ECAP signal 404 illustrates the voltage amplitude sensed as a result from a sub-detection threshold stimulation pulse. In other words, the stimulation pulse did not elicit a detectable ECAP signal in ECAP signal 404. Peaks 408 of ECAP signal 404 are detected and represent the artifact of the delivered stimulation pulse (e.g., a control pulse that may or may not contribute to a therapeutic effect for the patient). However, no propagating signal is detected after the artifact in ECAP signal 404 because the stimulation pulse was sub-detection threshold (e.g., the intensity of the stimulation pulse was insufficient to cause nerve fibers to depolarize and generate a detectable ECAP signal).

In contrast to ECAP signal 404, ECAP signal 406 represents the voltage amplitude detected from a supra-detection threshold stimulation pulse. Peaks 408 of ECAP signal 406 are detected and represent the artifact of the delivered stimulation pulse. After peaks 408, ECAP signal 406 also includes peaks P1, N1, and P2, which are three typical peaks representative of propagating action potentials from an ECAP. The example duration of the artifact and peaks P1, N1, and P2 is approximately 1 millisecond (ms). The time between two points in the ECAP signal may be referred to as a "latency" of the ECAP and may indicate the types of fibers being captured by the control pulse. ECAP signals with lower latency (i.e., smaller latency values) indicate a higher percentage of nerve fibers that have faster propagation of signals, whereas ECAP signals with higher latency (i.e., larger latency values) indicate a higher percentage of nerve fibers that have slower propagation of signals. Other characteristics of the ECAP signal may be used in other examples. Although stimulation amplitudes and ECAP amplitudes are generally described for the growth curves described here, the system may determine growth curves of latencies from the ECAP signals for different amplitudes or other different parameter values of the respective pulses from which the ECAP signals were sensed. In this manner, the system may analyze the growth curves of latencies with respect to parameter values changes to identify one or more parameter values to define subsequent stimulation pulses and/or a target latency to achieve during stimulation therapy.

The amplitude of the ECAP signal (e.g., of peaks within the ECAP signal) generally increases with increased amplitude of the stimulation pulse, as long as the pulse amplitude is greater than the threshold such that nerves depolarize and propagate the signal. The target ECAP characteristic (e.g., the target ECAP amplitude) may be determined from an ECAP signal associated with a neural response detected from pulses delivering therapy to patient 105 (FIG. 1). The ECAP signal thus is representative of the distance between the stimulation electrodes and the nerves appropriate for the stimulation parameter values of the pulses delivered at that time.

In some examples, processing circuitry 210 (FIG. 2) or other devices may be configured to determine a characteristic value for an ECAP signal, for example, from multiple different features of one or more signals associated with the ECAP signal. The characteristic value of the ECAP signal may be determined by removing an artifact from the ECAP signal using the processing circuitry. These different features may be incorporated into an average, weighted average, or other combination that represents the relative action potentials of the ECAP signal. Processing circuitry 210 may determine the characteristic value from different features of the same signal, such as the amplitude difference between two peaks in the ECAP signal and the amplitude difference between two different peaks in the ECAP signal. As another example of features from the same signal, processing circuitry 210 may determine the characteristic value based on an average of two different peaks in the second derivative signal. Alternatively, processing circuitry 210 may determine the characteristic value of the ECAP signal from features obtained from different signals. For example, processing circuitry 210 may determine the difference between the minimum and maximum values of the first derivative of the ECAP signal on either side of the P2 peak, determine the maximum value of the second derivative of the ECAP signal, and combine each of these factors into a single characteristic value of the ECAP signal. This single characteristic value of the ECAP signal may be referred to as a "composite" characteristic value because it is a composite of several different factors derived from the ECAP signal in order to obtain a more complete representation of the ECAP signal.

In one or more examples, the ECAP characteristic values may be determined after subtracting the artifact, to the extent an artifact may be present during some portion of the sensed ECAP signal. In some examples, that artifact may be modeled as a sum of exponential and a linear component. In another example, the artifact may be modeled sufficiently by either an exponential or a linear component alone. In order to fit the artifact to the response for the growth curve, several methods may be used. In one or more examples, the method may include estimating a minimum in the error function between the artifact model and the measured response. For example, if parameters of the function are P (e.g., time constant of the exponential, gain and linear slope and offset), the error function may be: $Err \left(P\right) = E \left[E\left(t\right)-A\left(P, t\right)\right]$

The optimal fit is to find Pₒₚₜ where the error Err(P) is minimized. The ECAP characteristic value may be determined the recording E(t) as: $ECAP \left(t\right) = E \left(t\right) - A \left({P}_{opt}, t\right)$

A common error function Err is something like a norm-2, which is defined as $E = sqrt \left[sum_t\left({\left(E\left(t\right)-A\left(P, t\right)\right)}^{2}\right.\right]$

An example model A(P, t) with four parameters is as follows: $A \left(P, t\right) = exp \left(-t/P\left(1\right)\right) * P \left(2\right) + t * P \left(3\right) + P \left(4\right)$

In one or more examples, the error function may be modified by a weight function W(t), where W(t) is high for instances where the neural response is low, for example, in the first region. For example, the W function may be high for t early in the measured waveform E(t) (e.g., prior to neural response developing), and low where the neural response can be high. In some examples, W(t) can be higher after the response. $E \left[P\right] = sqrt \left(sum_t\left(W\left(t\right)*{\left(E\left(t\right)-A\left(P, t\right)\right)}^{2}\right)\right.$

In this way, the model can be fit more specifically to the artifact, and not to the neural response, for example, for the first region. The weight can thus be adjusted by the system to reduce the effect of any stimulation artifact while maintaining the desired ECAP components of the signal. Note that, for this analysis, a uniform weight W may be used, so this feature may be optional.

It is also understood that, once the time constant P(1) is estimated, the rest of the parameters may be solved. For instance, in some examples, if M is defined as a matrix with rows [exp(-t/P(1)) t 1] and Wₘ is a matrix with diagonal equal to W, then parameters P(2) to P(4) may be: ${P}_{end} = \left(A′*diag\left(W′\right)*diag\left(W\right)*A\right) \ \left(A′*diag\left(W′\right)*diag\left(W\right)*data\right)$

In the above table, the "\" operator is a matrix inversion operator and diag(W) transforms the weight vector of length *n* to a matrix of weight *n* with contents of W. Features of interest in W may include high starting level (where most of the artifact is contained but ECAP response is low), and low weight for features which may contain the main ECAP energy (e.g. around samples 20 and 40). In addition, weight W might contain peaks corresponding to typical transition regions (e.g. peak around sample 11 or sample 30).

For real-time systems, the matrix multiplication operation may be fairly efficient. Thus, there may be an adaptive procedure to solve for P(1) (for example by back-propagation of error method) and then an analytic method to solve for P(2) to P(4). In one or more examples, if the artifact can change fairly rapidly, the speed of the back-propagation type of algorithm may be adjusted depending on the error term (e.g., large errors can lead to faster adaptation of P(1)). In some examples, either P(1) or range of P(1) can be estimated using equipment external to the implant, such as a clinician programmer or a patient programmer.

For certain weight functions, the equation for P_{end} can be a sparse equation and can be reduced to a non-FIR filter model. In addition, several P(1) candidates may be evaluated and the smallest one can be selected for the algorithm. Another alternative may be to determine evaluate several P(1) candidates and to pick the minimum one, but to utilize the adjacent near-by measurements to fit a curve, e.g. a parabola, to more precisely determine the location of the minimum. In this way, accuracy of the estimated neural response may be improved with fewer evaluations.

In one or more examples, the artifact may be removed from the ECAP using various methods, including, but not limited to, a standard method, artifact model method, high-pass filter method, or a correlation method, where each method uses the processing circuitry to determine the ECAP characteristic value.

In using the standard method (SM) to determine an ECAP characteristic value, waveforms Vᵢ(t) may be low-pass filtered (Kaiser filter, 11 tap, 4.5 kHz) to further band-limit and reduce asynchronous noise. In one or more examples, ECAP amplitude may be subsequently estimated (e.g., calculated) as a difference (e.g., in amplitude, such as in µV) between the P2 and N1 features of the ECAP. In one or more examples, N1 may be defined as the minimum amplitude of the filtered waveform in the temporal window from 0.3 to 0.6 milliseconds (ms), while P2 may be defined as the maximum amplitude in the temporal window from 0.7 to 1.1 ms. These windows of time may be set given the anticipated latencies and morphological characteristics of the ECAP. The latencies may be a function of the spacing between the stimulating and recording electrodes, along with the expected conduction velocity of ECAPs in the spinal cord. In case of a large artifact that starts positive and decays over time, it is possible that the N1 is greater than P2, where the N1-P2 may be computed to be negative.

The processing circuitry 210 may also, or alternatively, use an artifact model (AM) to determine a ECAP characteristic value. In one or more examples, the stimulation artifact may be composed of two decaying exponentials with different time constants. In one or more examples, over a relatively short post-stimulation window for estimating spinal ECAPS, for example, 1.5 ms, an artifact may be suitably modeled as the sum of a single exponential plus a linear component, which may more accurately estimate the ECAP amplitude. If Vᵢ(t) is the recorded voltage waveform after averaging, the estimate of artifact A(t) may be obtained by optimally fitting the following equation to data Vᵢ(t): $A \left(t\right) = {c}_{1} exp \left(-t/τ\right) + {c}_{2} t + {c}_{3}$

The fit may be performed by determining the minimum in the following error function over parameters c1, c2, c3, and τ: $E \left({c}_{1}, {c}_{2}, {c}_{3}, τ\right) = {\sum }_{t} {\left(V\left(t\right)-A\left(t\right)\right)}^{2}$

To solve this optimization problem, τ may be varied from 50 to 800 µs in 100 logarithmic steps. For each τ, E(τ) may BE determined by solving the following closed-form matrix equation: $M = \left⌊\begin{matrix}exp \left(-\frac{{t}_{0}}{τ}\right) & {t}_{0} & 1 \\ exp \left(-\frac{2 {t}_{0}}{τ}\right) & 2 {t}_{0} & 1 \\ … & … & …\end{matrix}\right⌋$ $C = \left(M′M\right) \ \left(M′V\right)$ $E \left(τ\right) = Norm \left(V-M C\right)$

In the above equation, t₀ may be the sampling period, C is a 3x1 vector of optimal c coefficients, V may be a vector composed of measured samples V(t), and Norm may represent a norm-2 operation. Optimal *τ* may be determined to be one that produced the smallest E(τ). The equation above was utilized to compute the C coefficients. After the artifact model is determined, the N1-P2 amplitude may be calculated or estimated from the denoised waveform V(t)-A(t) using the same N1 and P2 windows as in the standard method.

In one or more examples, the processing circuitry 210 may also, or alternatively, use a high-pass filter (HP) method. For example, the stimulation artifact may contain lower-frequency content relative to the ECAP in the later portion of the biopotential recording (e.g., greater than 0.6 ms after the end of the stimulation pulse). As such, another approach for mitigating the stimulation artifact overlapping the ECAP may be application of a high pass or differentiator filter. Such a filter may have the following benefits. The first peak response of the differentiator occurs at the high-slope transition of the ECAP from N1 to P2. This response may be delayed relative to N1, the first feature of the ECAP used by the SM to calculate the ECAP, and advantageously results in extra temporal isolation between the signal and the artifact with the differentiator. In addition, a simple differentiator may be implemented in a very computationally efficient manner, an important consideration for battery powered implantable medical devices.

A comb filter with response 1 - z⁻² may be utilized as a differentiator for the acquired biopotentials. After application of the differentiator filter, the waveform may be smoothed (Kaiser, FIR 11 tap filter; cutoff 4.5 kHz). The ECAP response may be computed as the difference between the maximum output in the temporal window from approximately 0.6 to 0.85 ms to the minimum output in the window from approximately 0.9 to 1.125 ms. The temporal windows may be set using similar considerations to those employed with the standard method.

The processing circuitry 210 may also, or alternatively, use a correlation method (CM) which estimates spinal cord activation by correlating the acquired biopotential with a synthesized filter template, T(t). Specifically, the neural response may be computed as: ${N}_{i} = {\sum }_{t} T \left(t\right) ∗ {V}_{i} \left(t-Δ\right)$

The template used here may have a mathematical expression of T(t) = B(t) sin(4πt/1.3)/N, where *t* is time in ms, *B(t)* is the Bartlett window, and *N* is the normalization factor, N = sum( B(t)2sin(4πt/1.3)2 ) over a 1.3-ms window, for example. The template may approximate the morphology of a typical ECAP signal. A duration of 1.3 ms may be used to optimize the match of the template with the observed response. The template may be orthogonal to the first three components of a Taylor expansion of the artifact waveform, namely the constant term, the linear term and the quadratic term. Thus, when the template is applied to a waveform containing both neural response and artifact, the artifact component may be reduced. However, variable latencies in neural responses routinely occur due to the differences in conduction velocities across subjects and delay in action potential initiation across stimulation levels or pulse width. The template may be matched to the neural recording and Fourier techniques may be utilized accordingly to compute the optimal delay, Δ. ${A}_{i} = {\sum }_{t} \frac{B \left(t\right) sin \left(4πt/1.3\right)}{N} ∗ {V}_{i} \left(t\right)$ ${A}_{r} = {\sum }_{t} \frac{B \left(t\right) cos \left(4πt/1.3\right)}{N} ∗ {V}_{i} \left(t\right)$ $Δ \left(ms\right) = - \left(\frac{∠ \left({A}_{i}+{A}_{R} 1j\right)}{4 π}\right) 1.3$

In some examples, to account for variability in neural response latencies, while avoiding non-physiological shifts in the response, the system may prevent Δ from decreasing below 0 or increasing above 0.18 ms.

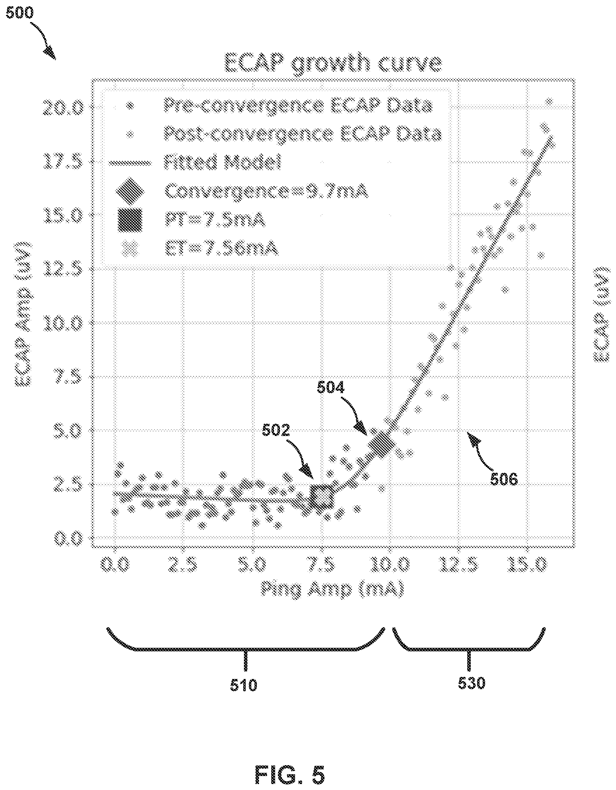

Once the ECAP characteristic value has been determined, the value may be used to determine an ECAP threshold, such as a neural threshold. A patient threshold of stimulation (for example, a perception threshold that represents the minimal stimulation current that causes a patient to feel the stimulation) may be correlated to the neural threshold. For example, FIG. 5 shows a relationship between the neural threshold and perception threshold level for various subjects. The neural threshold may be automatically calculated based on ECAP signals as described herein, and the determined perception thresholds and discomfort thresholds may be determined based on patient feedback to different stimulation amplitudes. As shown in FIG. 5, the determined paresthesia level for stimulation is tightly correlated to neural threshold 502. In other words, the neural threshold was determined to be very similar to the perception threshold. In one or more examples, a growth curve or a correlation curve may be developed that defines a relationship between ECAP characteristic values for different stimulation amplitudes (FIG. 5). Processing circuitry 210 may generate the growth curve by controlling stimulation circuitry to deliver stimulation pulses while sweeping the stimulation amplitude (e.g., iteratively increasing the amplitude) to sense respective ECAP signals and obtain ECAP characteristic values (e.g., data), which represents a neural response. In one or more examples, a storage device may store data which may define a correlation curve (e.g., a growth curve) defining a relationship between the ECAP characteristic values and stimulation amplitude. The system may determine the neural thresholds based on this correlation curve. The neural threshold may represent the estimated stimulation amplitude at which the patient response would transition from sub-perception, to perception of stimulation. The system may set an initial amplitude for stimulation based on the neural threshold or set a target ECAP value for therapy using the neural threshold (e.g., below, at, or above the neural threshold of the patient). In some examples, near the neural threshold of the patient, there may be a substantial curvilinear component, such as the beginning of an inflection portion of the correlation curve. In one or more examples, a non-physiologic component of the response can occasionally manifest below the neural threshold. In some examples, the response can grow linearly with increasing current and may be related to the residual artifact.

In one or more examples, as shown in FIG. 5, graph 500 includes growth curve 506 determined from the ECAP characteristic values (each dot in graph 500) from ECAP signals detected at the spinal cord. The dots lower on growth curve 506 and at amplitudes less than convergence point 504 (e.g., the diamond) represent ECAP characteristic values that were needed to determine ECAP threshold 502 which is the "X" along the smooth fitted line. In other words, once the system had delivered the pulses at the amplitude of convergency point 504 (e.g., 9.7 mA), no further ECAP characteristic values were needed to determine ECAP threshold 502. As shown ECAP threshold 502 is very similar to the blue box indicating the amplitude of pulses at which the patient identified the perception threshold. In some examples, the system can continue to deliver stimulation pulses part of the sweep after convergence point 504 and collect respective ECAP characteristic values that may be used to determine other ECAP thresholds, such as a discomfort threshold. Or, additional ECAP characteristic values may be used by the system to determine other metrics for the patient. In one or more examples, near the ECAP threshold 502 there may be a substantial curvilinear component. In some examples, determining the neural threshold is determined at least in part on a curvature of an inflection region of the growth curve 506. In some examples, a region after ECAP threshold 502 may be characterized by threshold (I*ₜₕᵣ*) and sigma (how fast response grows in this region). In one or more examples, a width of the curve relates to a therapeutic range of parameter settings offered to the patient and/or clinician.

In one or more examples, the following functional form may represent different regions of growth curve 506, for example, both the physiologic and artifact-driven, non-physiologic contributions to the ECAP growth curve 506: $E \left(I\right) = R \left(I, {I}_{thr}, σ\right) ⋅ {S}_{Resp} + I ⋅ {S}_{art} + N$ $R \left(I, {I}_{thr},σ\right) = \left(σ log\left(exp\left(-\frac{I - {I}_{thr}}{σ}\right)+1\right)+\left(I-{I}_{thr}\right)\right)$

In one or more examples, the estimate of neural activation, E(I), at a given stimulation current, I, may be the sum of three components. The components may include *R*(*I*, *Iₜₕᵣ*, *σ*) · *S_{Resp},* which captures the contribution of a neural response to the growth curve; *Sₐᵣₜ,* which describes a rate of growth of the artifact with current; and constant N, which is utilized to fit residual noise. The neural contribution may be characterized by parameters I*ₜₕᵣ, σ,* and *S_{Resp}.* I*ₜₕᵣ* represents the threshold for neural activation, while *σ* represents the spread, a parameter that defines how quickly the curve transitions between the curvilinear and linear region as stimulation current is increased. *Sᵣₑₛₚ* describes the rate of growth of neural response in the linear region. An example of the fit along with the parameters is shown FIG. 5A.

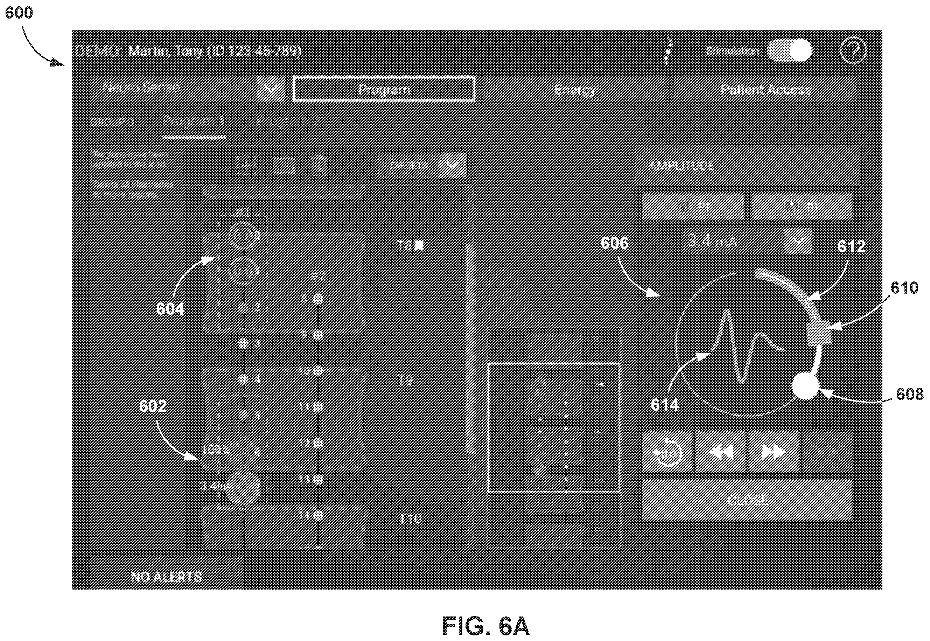

FIG. 6A is an example user interface 600 configured to receive user input and present information for determining a threshold from ECAP signals. User interface 600 may be an example of user interface 356 of external programmer 150 and be presented on a presence sensitive display. As shown in the example of FIG. 6A, user interface 600 includes various display and input fields. For example, a representation of electrodes (and leads in some examples) implanted within the patient may be provided. On this representation of the electrodes, user interface 600 may indicate the electrode combination 602 that is selected to deliver stimulation pulses and the sensing electrodes 604 selected to sense ECAP signals elicited by the pulses generated by electrode combination 602.

Parameter field 606 may be configured to receive user input and display information regarding one or more parameters that define stimulation pulses deliverable by electrode combination 602. For example, parameter field 606 includes limit input 608 which the user can move to set the maximum amplitude value that the system can deliver to the patient. Limit input 608 may be dragged by the user around a parameter value circle to set the maximum amplitude value. In other examples, the limit input 608 may be moved along a line or other shape.

Once the maximum amplitude value is set via limit input 608, the system may perform calibration of the ECAP threshold by delivering the sweep of pulses via electrode combination 602. Region 612 indicates the amplitude range that the sweep of pulses have already covered (e.g., pulses in that amplitude range have already been delivered). ECAP threshold 610 indicates the amplitude at which the ECAP threshold 610 was determined from the sweep of pulses. For example, ECAP threshold 610 may represent the neural threshold or perception threshold of the patient as determined automatically from the growth curve of the ECAP characteristic values determined from the pulses within amplitudes of region 612. In some examples, user interface 600 may receive adjustments to limit input 608 during the delivery of the sweep of pulses. However, once ECAP threshold 610 is determined, the system may grey out, lock, or otherwise change limit input 608 to indicate that no more adjustments are accepted or needed.

Display region 614 may present a representation of the ECAP signals received in response to delivery of the latest stimulation pulse. As shown in FIG. 6A, the representation in display region 614 may be green and represent a typical ECAP signal to indicate that the system is detecting ECAP signals appropriately. In other examples, a red spike or "V" may be shown to indicate that the system is only detecting artifacts or other information that is not the desired ECAP signals. The representation in display region 614 may be based on the morphology of the detected signal, the amplitude of the detected signal, and/or other aspects of the sensed signal.

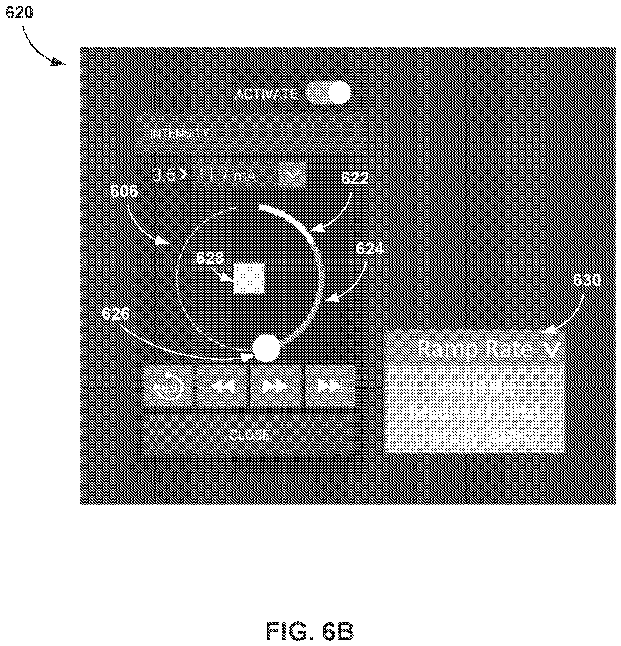

FIG. 6B is an example user interface 620 configured to receive user input selecting a ramp rate for stimulation pulse delivery during a sweep of amplitudes. User interface 620 may be an example, or part of, user interface 600. In the example of FIG. 6B, user interface 620 includes parameter field 606. That includes region 622, region 624, and limit input 608. Region 622 may be similar to region 612 in that pulses within the amplitudes (or intensity) of region 622 have already been delivered. Region 624 indicates the amplitudes of pulses of the sweep left to be delivered before limit input 626 that is movable by the patient to set the maximum amplitude value of the sweep of pulses. Stop input 628 may immediately stop delivery of stimulation during the sweep of pulses in response to user input selecting stop input 628.

In some examples, a clinician may desire to deliver stimulation pulses during the sweep using a pulse frequency the same as, or different from, the pulse frequency of stimulation therapy. Ramp rate selection field 630 may be configured to receive user selection of a pulse frequency of the stimulation pulses during the sweep of pulses. In some examples, lower pulse frequencies may yield larger ECAP signals and less patient perception. However, it may take longer to complete the sweep of pulses using a lower pulse rate. In one example, therapy pulse frequency may be 50 Hz, but other lower selectable pulse frequencies may include a medium rate of 10 Hz and a low rate of 1 Hz. Additional selectable pulse frequencies may be provided in some examples, or different pulse rates may be used. In some examples, the ramp rate selection field 630 may be configured to receive numerical input from the user specifying the pulse rate for the sweep of pulses.

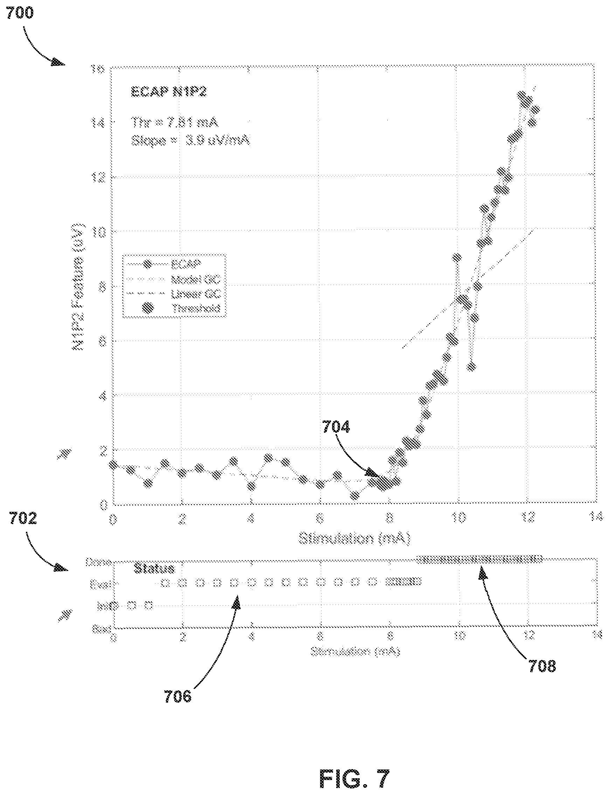

FIG. 7 is an example growth curve 700 of ECAP characteristic values and real-time status of the threshold based on available ECAP characteristics as stimulation pulses are delivered. As shown in the example of FIG. 7, each dot in growth curve 700 may be an ECAP characteristic value determined from a respective ECAP signal. The system may generate each dot in response to the ECAP characteristic value being determined and track the progress and attempt to determine the ECAP threshold based on the ECAP characteristic values already determined.

Status field 702 indicates each point in time of the status of the ECAP threshold determination from the received ECAP characteristic values, or progress of growth curve 700. Evaluation squares 706 indicate that the system is still evaluating the ECAP threshold using the ECAP characteristic at that time in growth curve 700 above. As soon as the system determines the ECAP threshold 704, status field 702 shows done squares 708 indicating that those ECAP characteristic values are no longer needed because the ECAP threshold 704 as been determined. Generally, one or more ECAP characteristic values may be determined beyond the amplitude of ECAP threshold 704 in order to determine when ECAP threshold 704 occurred. As discussed above, as soon as the system determines that no more ECAP characteristic values are needed to determine the ECAP threshold 704, the system can abort the sweep of pulses.

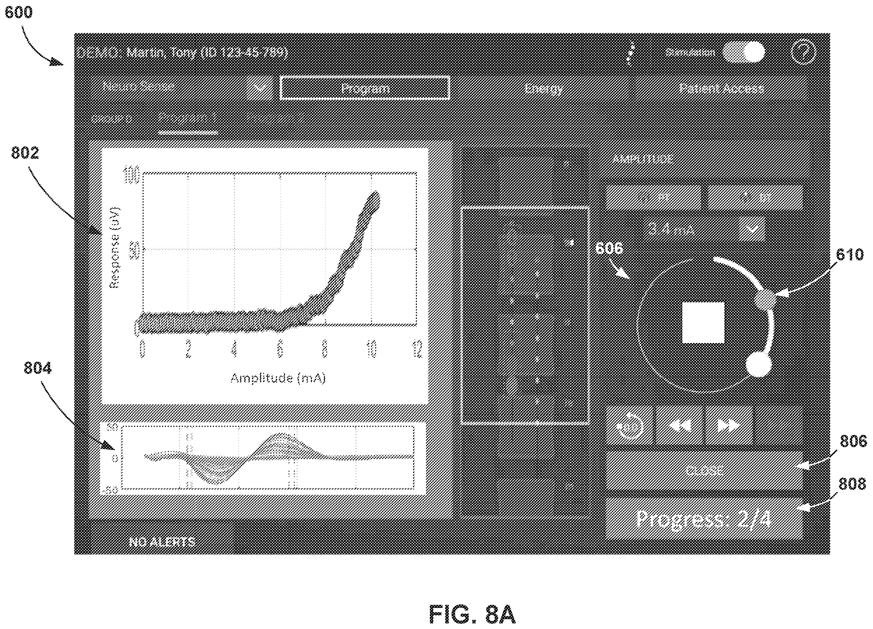

FIG. 8A is an example user interface 600 configured to receive user input and present information regarding sensed ECAP signals and determined ECAP characteristics. As shown in FIG. 8A, user interface 600 may display parameter field 606 and determined ECAP threshold 610. In addition, user interface 600 may display growth curve 802 as the collection of ECAP characteristic values determined from the stimulation pulses in the sweep of pulses. ECAP waveforms 804 include representations of each ECAP signal sensed by the system from respective stimulation pulses of the sweep of pulses. When selected by the user, close button 806 may stop the sweep of pulses and close the user interface 600. Progress indicator 808 indicates the progress of determination of ECAP threshold 610. In some examples, progress indicator 808 may indicate how many electrode combinations have been used to deliver a sweep of pulses out of the schedule number of different electrode combinations.

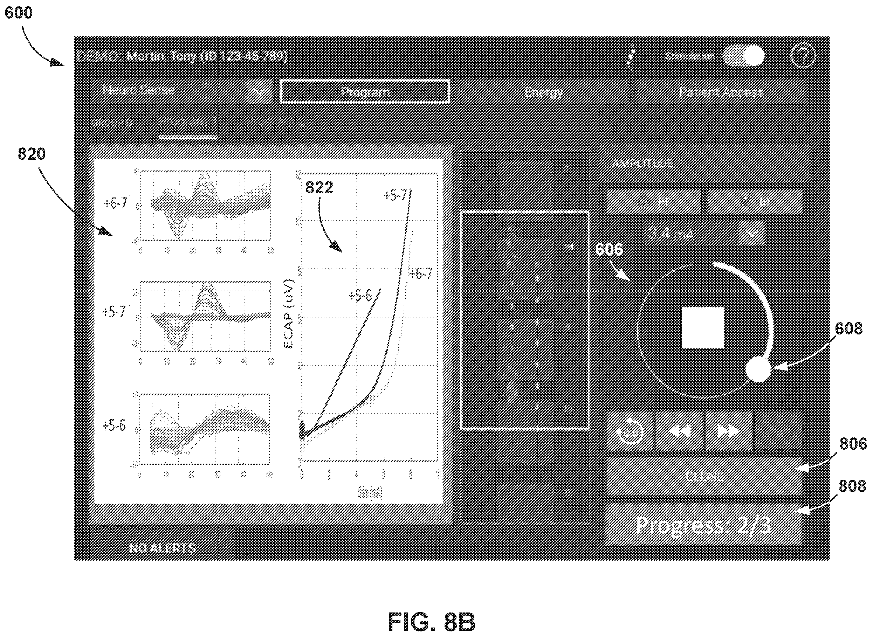

FIG. 8B is an example user interface 600 configured to receive user input and present information regarding sensed ECAP signals and growth curves for multiple electrode combinations. As shown in FIG. 8B, user interface 600 may display parameter field 606 and limit input 608. In addition, user interface 600 may display ECAP waveforms 820 and growth curves 822. ECAP waveforms 820 include representations for all of the ECAP signals sensed by the system for each of the three different electrode combinations from which stimulation pulses have been delivered. Similarly, growth curves 822 indicate the growth curve for each of the three electrode combinations from which the stimulation pulses have been delivered.

In some examples, the system may deliver the complete sweep of pulses (or at least the pulses of the sweep needed to determine the ECAP threshold) from one electrode combination before moving to the next electrode combination. In other examples, the system may interleave the pulses from each electrode combination such that the sweeps of pulses for the electrode combinations are performed concurrently. In this example, the system may update ECAP waveforms 820 and growth curves 822 for each of the electrode combinations as the pulses are delivered. In other examples, each of growth curves 822 may be determined by sensing ECAP signals from all of the respective electrode combinations simultaneously or at least from the same delivered stimulus. In this manner, the system may record the sensed ECAP signals at the multiple potential electrode combinations at the same time and in response to the same delivered pulse. The system may continue with one or more sweeps from respective electrode combinations to determine the respective ECAP thresholds even if the ECAP threshold for one electrode combination has been determined. ECAP waveforms 820 and growth curves 822 may assist the clinician in determining which electrode combination to use for stimulation therapy. In other examples, the system may stop the collection of ECAP signals (and associated stimuli) for growth curve generation in response to determining the inflection point of the growth curve or otherwise collecting sufficient information around the efficacy of that particular electrode combination. In this manner, the system may automatically terminate the sweep of pulses as soon as sufficient data is collected in order reduce the likelihood of delivering stimulus with an amplitude greater than the discomfort threshold of the patient.

When selected by the user, close button 806 may stop the sweep of pulses and close the user interface 600. Progress indicator 808 indicates the progress of determination of ECAP threshold 610. In some examples, progress indicator 808 may indicate how many electrode combinations have been used to deliver a sweep of pulses out of the schedule number of different electrode combinations.

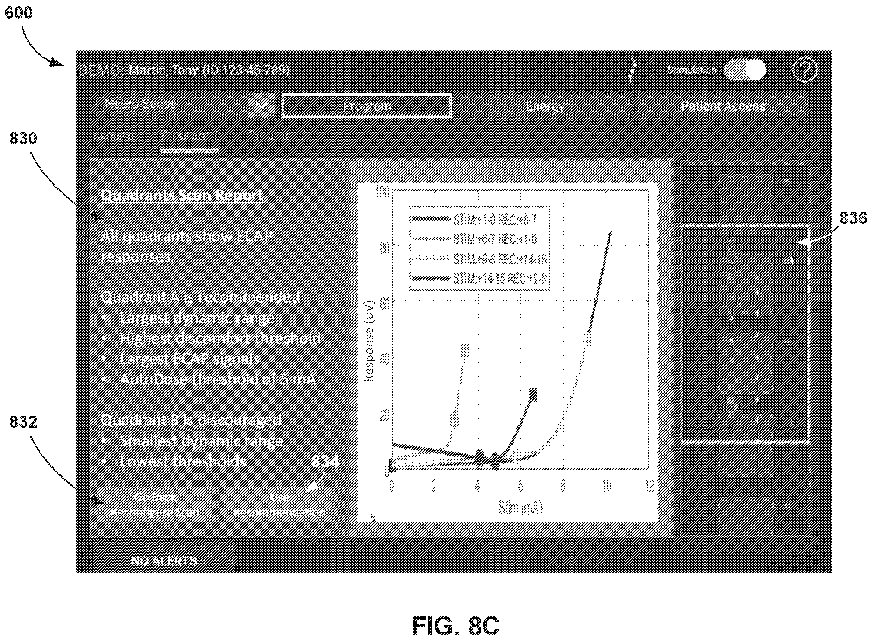

FIG. 8C is an example user interface 600 configured to present a summary of determined thresholds and recommendations based on received ECAP signals for multiple electrode combinations. As shown in FIG. 8C, summary field 830 includes information associated with the sweep of pulses delivered for each electrode combination and the ECAP thresholds determined. In the example, the ECAP thresholds include both the perception threshold and the discomfort threshold. Summary field 830 may include the growth curves for each electrode combination in addition to the ECAP thresholds determined for each growth curve. Electrode combination field 836 may indicate the electrode combination from the available electrodes that is recommended according to the result of the ECAP threshold determination for the tested electrode combinations. Go back button 832 may case user interface 600 to return to a previous screen, and approve button 834 may, when selected, cause the system to accept the recommended parameter values for stimulation, such as the electrode combination, amplitude values, and/or ECAP thresholds.

In some examples, summary field 830 may include additional information for the clinician. This additional information may include information such as which electrode combination (or quadrant) that is recommended and/or which electrode combination is discouraged. Reasons for recommendation may include the electrode combination with the largest dynamic range (e.g., the largest amplitude between the perception threshold and the discomfort threshold), the highest discomfort threshold, the largest ECAP signals (e.g., easier to detect), and/or a detectable ECAP threshold. Reasons for discouraging an electrode combination may include the smallest dynamic range between thresholds and/or the lowest threshold that may be more difficult to detect. In addition, summary field 830 may include recommendations for initial stimulation parameter values, target ECAP characteristic values for feedback, and/or increment or decrement levels for adaptive stimulation based on subsequently detected ECAP characteristics.

External programmer 150 may operate as a patient programmer that may receive input from the patient as a part of the calibration of stimulation, such as delivering the sweeps of pulses and selecting stimulation parameters. For example, the programmer may be configured to receive a patient's response during the sweep of pulses, such as receiving input indicating when a pulse is perceived (e.g., a perception threshold) and/or when the pulses are uncomfortable (e.g., a discomfort threshold). In some examples, the patient's input identifying the perception threshold may not exactly match the automatically determined ECAP threshold from the sweep of pulses. In this manner, the system may determine an offset amplitude corresponding to the difference between the ECAP threshold and the patient's input determined perception threshold. The system may apply this offset amplitude to detected ECAP characteristic values for better detection of ECAP characteristic values related to thresholds or target values later in therapy.

The programmer may receive user input identifying events for later review (e.g., high stimulation, or lack of therapy) which store the ECAP waveforms or other information around that event in time. The programmer can then upload the stored data at a later time. When a sweep of pulses is active, the programmer may be configured to receive patient input requesting the system to abort the sweep of pulses. The programmer may provide a limited set of input fields during a sweep of pulses for simplicity, such as a "I felt it" button and a "stop" button to enable the patient to easily control the system during the sweep of pulses.

In the calibration session in which the sweep of pulses is provided, different scenarios may occur for different patients. For example, the system may determine that the newly determined ECAP thresholds do not match the old thresholds and recommend use of the new ECAP thresholds. The system may require patient approval to switch to the new ECAP thresholds. Of ECAPs used to be detectable, but no ECAP signals were detected during the sweep of pulses, the system may recommend that the patient schedule a visit with the clinician. In some examples, the programmer may instruct the patient to assume and maintain a certain posture (e.g., lying down or sitting down) during the sweep of pulses. In some examples, the programmer may request that sweeps be performed in different postures and determine different ECAP thresholds for each posture.

In some examples, the system may take action in response to sensing ECAP signals where ECAP signals should not be detectable. Some therapies may include stimulation pulses (singular or groups of pulses) that are intended to have an intensity below perception threshold of the patient. If the system determines that ECAP signals are detected, or ECAP characteristics are detected above the ECAP threshold (e.g., the perception threshold), the system may provide an alert to the patient and/or clinician that stimulation parameter values for the therapy may need to be reviewed. In some examples, the system may automatically withhold or terminate the stimulation therapy until the patient or clinician request to resume therapy and/or one or more parameter values are adjusted until ECAP signals are again below the ECAP threshold.

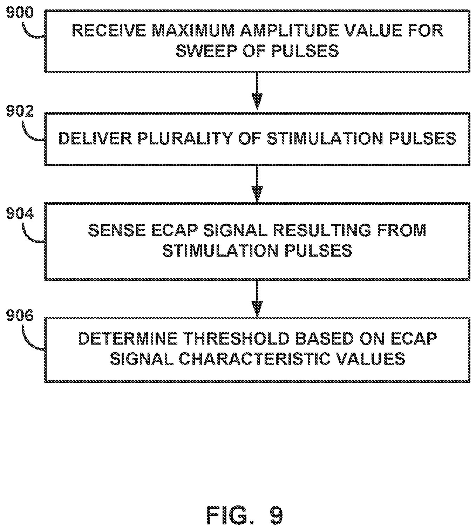

FIG. 9 is a flow diagram illustrating an example technique for determining a threshold based on ECAP signal characteristic values. IMD 200 and processing circuitry 210 will be described in the example of FIG. 9, but other IMDs, such as IMD 110, or other devices (e.g., external programmer 150) or systems may perform, or partially perform, the technique of FIG. 9.

In one or more examples, processing circuitry 210 receives a maximum amplitude value for a sweep of pulses (900). For example, processing circuitry 210 receives user input setting the maximum amplitude value for the sweep of pulses. Processing circuitry 210 then controls IMD 200 to deliver a plurality of stimulation pulses as the sweep of pulses (902). In some examples, each stimulation pulse of the plurality of stimulation pulses is at least partially defined by a different respective value of a stimulation parameter. For example, the pulses may have an iteratively increasing amplitude value. In this manner, the different values of the stimulation parameter may constitute a sweep of increasing stimulation amplitudes that may be linear, non-linear, adaptive based on feedback, and/or some combination thereof.

The processing circuitry 210 may also control IMD 200 to sense the respective ECAP signals resulting from the stimulation pulses (904). In some examples, the method may include receiving, by the processing circuitry, evoked compound action potential (ECAP) signal information, wherein the ECAP signal information comprises ECAP signals sensed by sensing circuitry and elicited by the plurality of stimulation pulses, and determining, by the processing circuitry and based on the ECAP signal information, ECAP characteristic values for each of the ECAP signals elicited by the plurality of stimulation pulses. In some examples, the ECAP characteristic value may comprise a direct measurement, by processing circuitry, between an N1 peak and a P2 peak of the ECAP signal information. In some examples, the ECAP characteristic values may comprise the ECAP signal information with an artifact removed therefrom. In one or more examples, removing the artifact may include modeling, for example by the processing circuitry, the artifact as a sum of a single exponential component plus a linear component, and removing the sum from each ECAP signal. In yet another example, the artifact may be sufficiently modeled solely as a linear component or exponential. In some examples, modeling the artifact by the processing circuitry includes estimating a minimum of an error function by weighting the error function higher in a first region than in a second region, where the first region is prior to a patient neural response and the second region is after the patient neural response. In one or more examples, removing the artifact includes passing the ECAP signal through a high-pass filter.

The method may further include determining, for example by processing circuitry 210 and based on the ECAP characteristic values, the ECAP threshold (e.g., a neural threshold) of the patient (906). In some examples, the process of sensing the ECAP signals may include measuring the ECAP signals for a growth curve of a patient while the patient remains in the same position. In some examples, a correlation curve defines a relationship between ECAP characteristic values and stimulation amplitude, where the system can determine a neural threshold based on the correlation curve (e.g., the growth curve). In some examples, the method may include storing data on a storage device 212, the data defining a correlation curve defining a relationship between ECAP characteristic values and stimulation amplitude, where the correlation curve includes a first region where change in amplitude is defined in part by residual artifact, and a second region where change in amplitude is defined in part by patient neural response, where the first region is prior to the neural threshold and the second region is after the neural threshold. In some examples, determining the neural threshold includes at least determining the neural threshold at least in part on a curvature of an inflection region of the curve.

In some examples, the relationship of the sensed ECAP signal to the stimulation amplitude can be defined by: $E \left(I\right) = R \left(I, {I}_{thr}, σ\right) ⋅ {S}_{Resp} + I ⋅ {S}_{art} + N ;$ where: $R \left(I, {I}_{thr},σ\right) = \left(σ log\left(exp\left(-\frac{I - {I}_{thr}}{σ}\right)+1\right)+\left(I-{I}_{thr}\right)\right) ;$
E(I) comprises the estimated neural response at a given stimulation current I;
*Iₜₕᵣ* comprises an ECAP threshold;
*σ* comprises a parameter defining a rate of transition between a linear region of data and a curved region of data;
*Sₐᵣₜ* comprises is a rate of growth of an artifact with current; and
*Sᵣₑₛₚ* comprises a rate of growth in the linear region of data.

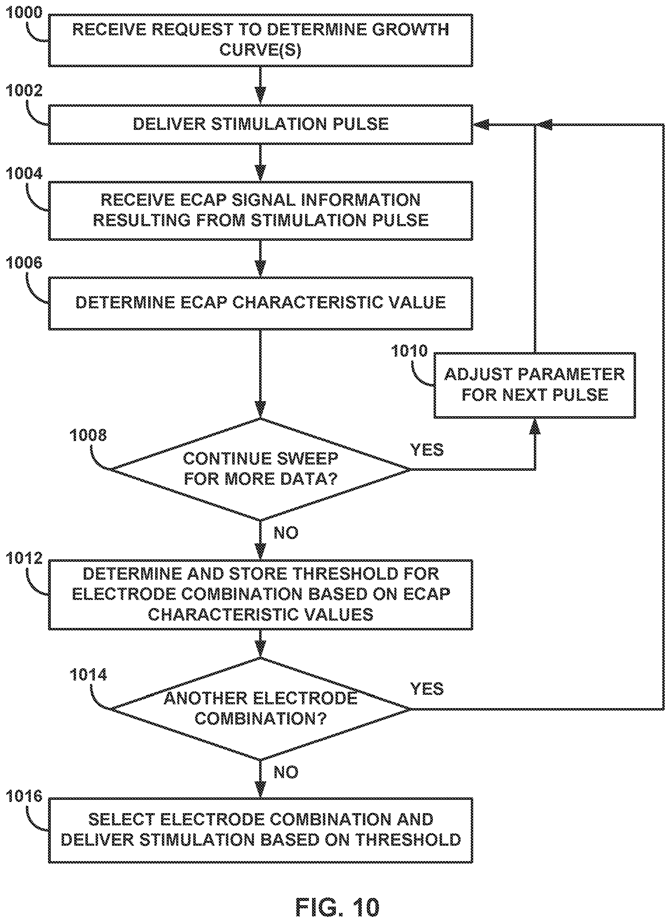

FIG. 10 is a flow diagram illustrating an example technique for determining a threshold for one or more electrode combinations through a sweep of pulses. IMD 200 and processing circuitry 210 will be described in the example of FIG. 10, but other IMDs, such as IMD 110, or other devices or systems may perform, or partially perform, the technique of FIG. 10.

In the example of FIG. 10, processing circuitry 210 receives a request to determine one or more growth curves for respective electrode combinations (1000). Then, processing circuitry 210 controls IMD 200 to deliver a stimulation pulse (1002). The stimulation pulse is at least partially defined by a respective value of a stimulation parameter. The processing circuitry 210 may also control IMD 200 to sense ECAP signals resulting from the stimulation pulse. In some examples, processing circuitry 210 may receive, by the processing circuitry 210, evoked compound action potential (ECAP) signal information (1004). In one or more examples, the ECAP signal information may include ECAP signals sensed by sensing circuitry and elicited by the stimulation pulse.

Processing circuitry 210 then determines ECAP characteristic value(s) for the ECAP signals elicited by the stimulation pulses, where the ECAP characteristic value(s) are based on the ECAP signal information (1006). In some examples, the ECAP characteristic value may comprise a direct measurement, by processing circuitry 210, between an N1 peak and a P2 peak of the ECAP signal information. In some examples, the ECAP characteristic values may comprise the ECAP signal information with an artifact removed therefrom.

The processing circuitry 210 then determines whether to continue with a sweep of different parameter values if more data is needed to determine the ECAP threshold (1008). The sweep may include iteratively increasing a stimulation parameter value, such as an amplitude, for successive stimulation pulses. The processing circuitry 210 may determine to continue to sweep if the parameter value is not yet at a predetermined value, if a neural threshold cannot be determined from already collected ECAP characteristic values, or if processing circuitry 210 has not received patient input requesting to stop the sweep. The processing circuitry 210 may stop the sweep in response to the parameter value reaching the predetermined value, in response to determining that the neural threshold can be determined, or in response to receiving patient input requesting to stop the sweep (e.g., when stimulation amplitude has reached a discomfort threshold). In some examples, if the amplitude exceeds the maximum amplitude value set by the user, processing circuitry 210 may stop the sweep. If processing circuitry 210 determines to continue the sweep ("YES" branch of block 1008), processing circuitry 210 adjusts the parameter value (e.g., increases the stimulation amplitude) for the next pulse (1010) and continues to control IMD 200 to deliver the next stimulation pulse (1002).

If processing circuitry 210 determines to stop the sweep ("NO" branch of block 1008), processing circuitry 210 determines the ECAP threshold (e.g., the neural threshold) of a patient for the electrode combination and based on ECAP characteristic values (1012). For example, the method may further include determining, for example, by processing circuitry 210 and based on the ECAP characteristic values, a neural threshold of the patient that may correspond to the perception threshold of the patient.

In some examples, processing circuitry 210 senses ECAP signals for measuring a growth curve of a patient while the patient remains in the same position. In some examples, the IMD 200 or the system 100 may detect portions of time when no motion is occurring, for example, by use of an artifact or a sensor such as an accelerometer. In some examples, a correlation curve defines a relationship between ECAP characteristic values and stimulation amplitude, where the correlation curve is used for the growth curve to determine a neural threshold. In some examples, the method may include storing data on a storage device 212, the data defining a correlation curve defining a relationship between ECAP characteristic values and stimulation amplitude, where the correlation curve includes a first region where change in amplitude is defined in part by residual artifact, and a second region where change in amplitude is defined in part by patient neural response, where the first region is prior to the neural threshold and the second region is after the neural threshold. In some examples, determining the neural threshold is determined at least in part on a curvature of an inflection region of the curve.

In some examples, the relationship is defined by: $E \left(I\right) = R \left(I, {I}_{thr}, σ\right) ⋅ {S}_{Resp} + I ⋅ {S}_{art} + N ;$ where: $R \left(I, {I}_{thr},σ\right) = \left(σ log\left(exp\left(-\frac{I - {I}_{thr}}{σ}\right)+1\right)+\left(I-{I}_{thr}\right)\right) ;$
E(I) comprises the estimated neural response at a given stimulation current I;
*Iₜₕᵣ* comprises an ECAP threshold;
*σ* comprises a parameter defining a rate of transition between a linear region of data and a curved region of data;
*Sₐᵣₜ* comprises is a rate of growth of an artifact with current; and
*Sᵣₑₛₚ* comprises a rate of growth in the linear region of data.

Processing circuitry 210 then determines if there is another electrode combination for which an ECAP threshold needs to be determined (1014). If there is another electrode combination ("YES" branch of block 1014), processing circuitry 210 selects the next electrode combination and again delivers stimulation pulses for the sweep of the next electrode combination (1002). In other examples, processing circuitry 210 may control the system to interleave stimulation pulses and respective different electrode combinations sensing ECAP signals with each other so that the sweeps for different electrode combinations occur concurrently. In some examples, processing circuitry 210 may control the system to record ECAP signals from multiple sensing electrode combinations at the same time an in response to the same stimulation pulse. In this manner, the growth curves may be generated more quickly and from stimuli delivered with the same patient conditions. If there is no other electrode combination to review ("NO" branch of block 1014), processing circuitry 210 then selects the electrode combination based on the respective ECAP thresholds and then delivers stimulation pulses based on the ECAP threshold (1016). For example, processing circuitry 210 may set the initial amplitude values to the ECAP threshold or determine a target ECAP value based on some percentage or multiplier of the ECAP threshold. Once the ECAP threshold has been determined, processing circuitry 210 may deliver and/or adjust stimulation pulses.

In other examples, processing circuitry 210 may select more than one electrode combination, such as some or all electrode combinations, for sensing ECAP signals. The multiple electrode combinations may be based on the growth curves. Processing circuitry 210 may use the growth curves from the selected electrode combinations to determine an average, a minimum, a maximum, or other ECAP threshold and monitor the signals from the electrode combinations to use for feedback and controlling subsequent stimulation.

Based on the ECAP characteristic value and neural threshold, processing circuitry 210 can determine a parameter value for subsequent electrical stimulation pulses. For example, if the ECAP characteristic value is above or below a target characteristic value, processing circuitry 210 may reduce or increase, respectively, the value of a parameter that defines subsequent stimulation pulses. In one or more examples, processing circuitry 210 uses a target ECAP characteristic value associated with a percentage above or below the neural threshold. Processing circuitry 210 then controls stimulation circuitry to deliver electrical stimulation at least partially defined by the adjusted value of the parameter. For example, the parameter may be a current amplitude or pulse width of the stimulation pulses. Processing circuitry 210 may continue to perform the process of FIG. 10 in a loop to continually use characteristic values of ECAP signals as feedback (e.g., one or more thresholds and/or targets) for adjusting stimulation pulses in a closed-loop manner.

In some examples, processing circuitry 210 may be configured to sense ECAP signals in response to therapeutic pulses that are part of therapy. In other examples, processing circuitry 210 may be configured to sense ECAP signals from control pulses that are configured to elicit detectable ECAP signals and may be interleaved with informed pulses. The informed pulses may be configured to provide a therapeutic effect for the patient, but the system may not be configured to detect ECAP signals elicited, if any, from the informed pulses. Some informed pulses may be pulses that are delivered with a frequency too high for the system to detect ECAP signals between subsequent pulses. In some of these cases, the system may deliver trains of these high frequency pulses (or continuous signals such as a sine wave), but processing circuitry 210 may be configured to only attempt top sense an ECAP signal elicited from the last pulse in the high frequency pulse train (or the end of the continuous signal). High frequency pulse trains may have frequencies greater than 100 Hz, greater than 200 Hz, greater than 500 Hz, greater than 900 Hz, greater than 1,000 Hz, or even higher frequencies. In some examples, processing circuitry 210 may be configured to detect the ECAP signal after the last pulse in a pulse train when the inter-pulse quiescent period (e.g., the period of time between the end of one pulse and the start of the next pulse) is less than the need time to sense an ECAP signal (e.g., an inter-pulse quiescent period greater less than 1 millisecond, less than 500 microseconds, or less than 200 microseconds).

The example of FIG. 10 is described for the purpose of determining an ECAP threshold for different electrode combinations. However, in other examples, the system may perform sweeps of pulses for other parameter variations. For example, the system may perform sweeps of pulses for different pulse widths, pulse frequencies, or even recharge modes. For example, each stimulation pulse may be followed by a passive recharge pulse (e.g., passive current drain from tissue), active recharge pulse (e.g., bi phasic pulses), or some combination thereof, to equalize charge at the tissue following delivered stimulation pulses. For example, one sweep may be performed for pulses followed by passive recharge pulses, one sweep may be performed for pulses followed by active recharge pulses, and another sweep may be performed for pulses that have an active recharge pulse that is half the pulse width of the stimulation pulses (meaning that half the remaining charge is dissipated by the active recharge pulse) with the remaining charge dissipated by passive recharge. The system may identify the ECAP threshold or other aspect of the growth curves to determine which type of recharge, active, passive, or a combination of both, may benefit patient therapy and the system. Since passive recharge consumes less battery power, the system may select full passive recharge or partial passive recharge as long as the ECAPs are still detectable and sufficient for closed-loop therapy. In some examples, the system may have threshold values for switching the passive recharge. For example, when stimulation pulse amplitudes are less than an amplitude threshold of 2 mA, the system may try passive recharge since there is less charge in the tissue to dissipate, which could be achieved using passive recharge. In another example, when ECAP amplitudes are above a certain threshold, such as 10 micro Volts, for example, the system may test passive recharge because the ECAP signals may still be detectable even if the ECAP signal arrives when the passive recharge is not yet complete.

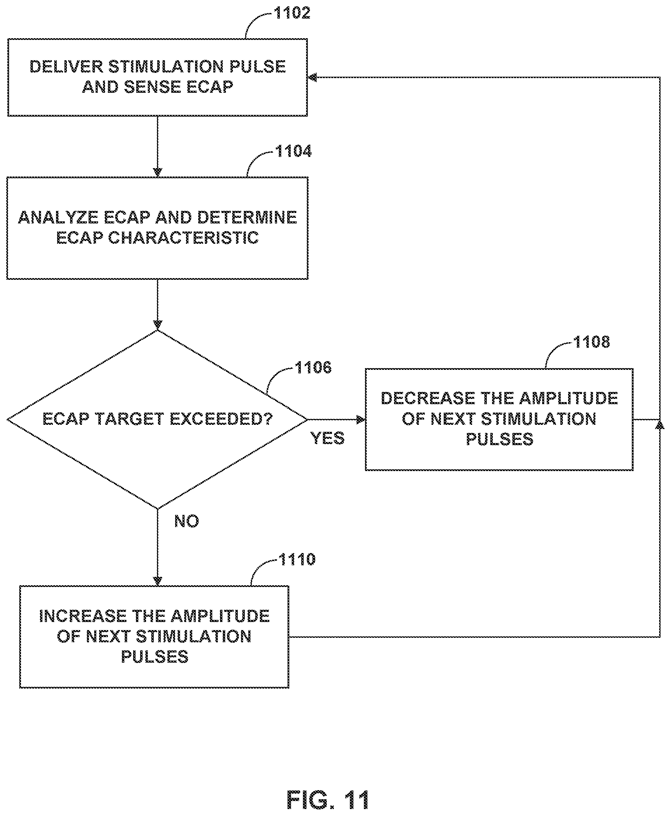

FIG. 11 is a flow diagram illustrating an example technique for determining an ECAP threshold and controlling electrical stimulation. For convenience, FIG. 11 is described with respect to IMD 200 of FIG. 2. However, the technique of FIG. 11 may be performed by different components of IMD 200 or by additional or alternative devices. The technique of FIG. 11 is an example feedback mechanism for controlling stimulation therapy using sensed ECAP signals.

As illustrated in FIG. 11, processing circuitry 210 of IMD 200 delivers a stimulation pulse and senses the resulting ECAP elicited by the stimulation pulse (1102). Processing circuitry 210 receives and analyzes the ECAP to determine an ECAP characteristic value (1104). The processing circuitry 210 evaluates whether the ECAP characteristic value has exceeded the target ECAP value (1106). The target ECAP value may be based on the ECAP threshold determined prior using the sweep of pulses. In some examples, the processing circuitry 210 may target a lesser percentage than the ECAP characteristic value associated with the ECAP threshold, for example to extend battery life of IMD 200. For example, processing circuitry 210 may target 70% of the ECAP threshold. In some examples, the ECAP threshold target may include a range of values. In one or more examples, the ECAP threshold target may include a range of 30% of the ECAP threshold to an upper limit of below a discomfort threshold for a patient.

If processing circuitry 210 determines that the representative amplitude of the one or more ECAP signals is greater than the target ECAP value ("YES" branch of block 1106), processing circuitry 210 decreases the amplitude of the next stimulation pulses (1108). For example, the amplitudes of the stimulation pulses may be decreased by predetermined steps. As another example, the respective amplitudes of the stimulation pulses may be decreased by an amount proportional to the difference between the representative amplitude and the ECAP characteristic value associated with the neural response. If processing circuitry 210 determines that the representative characteristic value is less than the ECAP characteristic value for the target ECAP value, ("NO" branch of block 1106), processing circuitry 210 moves to block 1110.

At block 1110, processing circuitry 210 increases the amplitude of the stimulation pulses by an amount proportional to the difference between the representative amplitude and the target ECAP characteristic value. Processing circuitry 210 then continues to deliver a stimulation pulse according to the increased or decreased amplitudes. In some examples, the decrease or increase applied to the stimulation pulses in steps 1108 or 1110, respectively, may apply to the amplitude or another parameter of the next-scheduled stimulation pulse. In this manner, even if a decrease is applied to the next stimulation pulse, the overall new amplitude of the next stimulation pulses may still be greater than the previous stimulation pulse if the scheduled amplitude of the next stimulation pulse minus the decrease is still greater than the amplitude of the previous stimulation pulse.

Although the process of FIG. 11 is described for adjusting the amplitude of the stimulation pulses (e.g., control pulses and/or stimulation pulses), other parameter values may be changed in other examples. For example, sensed ECAP signals may be used to increase or decrease the pulse width of the stimulation pulse to adjust the amount of charge delivered to the tissue to maintain consistent volume of neural activation. In other examples, electrode combinations may be adjusted in order to deliver different amounts of charge and modify the number of neurons being recruited by each stimulation pulse. In other examples, processing circuitry 210 may be configured to adjust the pulse rate or duty cycle of the stimulation pulses.

In some examples, therapy, such as for SCS stimulation, may be programmed. For example, setting parameter values for therapy may be based on a patient sensory threshold. In some examples, the programming and/or closed-loop control of SCS stimulation may be based on the ECAP threshold, including the techniques for estimating neural threshold described herein. In one or more examples, determination of the estimated neural threshold may be performed by the patient. For example, the patient may be asked to stay in a certain position, for example with the patient programmer 150 (FIG. 1), and then growth curves would be measured by processing circuitry using the techniques described herein, and the ECAP threshold would be determined. In some examples, if stimulation therapy becomes uncomfortable, the patient may terminate the stimulation.

In some examples, a configuration for measurement would be selected to facilitate a larger response, which may be different than one used for ECAP therapy. The above-described steps can be repeated by the patient to optimize therapy in various positions in combination with the position-sensor technology.

Once the ECAP threshold is determined for each component of the program, stimulation parameters of a SCS stimulation program may be determined based on the ECAP threshold. For example, amplitude level for stimulation pulses of each program can be set as a percentage of the estimated neural threshold (e.g., 65%). Alternatively, both neural thresholds and sigma can be utilized to estimate the stimulation levels. For example, stimulation can be set to neural threshold plus 1/sigma to get a nearly constant response.

In some instances, one can record changes in threshold in the presence of other stimulation (e.g., high-rate stimulation) and compare to lower-rate stimulation to determine proper dosing of higher-rate stimulation. In some examples, real-time measurements of ECAP signals may be used to determine ECAP characteristic values for the ECAP signals, and ECAP thresholds may be determined. The real-time determination of ECAP thresholds may be utilized to set stimulation levels. For example, occasional measurements near a sensation threshold can be utilized to measure threshold and establish a "dose" (e.g., intensity, duration, etc.) for other components of stimulation. Alternatively, when a position of the person is changed, one can adjust the stimulation automatically based on best neural threshold.

The techniques described in this disclosure may be implemented, at least in part, in hardware, software, firmware or any combination thereof. For example, various aspects of the described techniques may be implemented within one or more processors or processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also perform one or more of the techniques of this disclosure.

Such hardware, software, and firmware may be implemented within the same device or within separate devices to support the various operations and functions described in this disclosure. For example, processing circuitry may conduct processing off-line and conduct automatic checks of patient ECAP signals and update programming from a remote location. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

The present invention is defined by the appended claims.

## Claims

1. A system comprising:
processing circuitry (210) configured to:
receive a maximum amplitude value for a plurality of stimulation pulses;
control stimulation generation circuitry to begin delivery of the plurality of stimulation pulses at an initial amplitude value less than the maximum amplitude value and iteratively increase a subsequent amplitude value of a next stimulation pulse of the plurality of stimulation pulses up to the maximum amplitude value;
receive, for each stimulation pulse of the plurality of stimulation pulses, evoked compound action potential (ECAP) signal information, wherein the ECAP signal information comprises ECAP signals sensed by sensing circuitry;
determine, from the ECAP signal information for each stimulation pulse, ECAP characteristic values for each of the ECAP signals; and
determine, based on the ECAP characteristic values and during the delivery of the stimulation pulses with iteratively increasing amplitude values, at least one threshold from which one or more stimulation parameter values for subsequent stimulation pulses are determined.

2. The system of claim 1, wherein the processing circuitry (210) is configured to:
receive the maximum amplitude value via a user interface;
control the user interface to display a representation of a range of amplitude values for the plurality of stimulation pulses deliverable during a calibration routine;
receive, via the user interface, user input specifying the maximum amplitude value; and
control the user interface to display a representation of the at least one threshold.

3. The system of any of claims 1 and 2, wherein the processing circuitry (210) is configured to present the at least one threshold on the representation of the range of amplitude values.

4. The system of any of claims 1 through 3, wherein the processing circuitry (210) is configured to:
generate a graph of the ECAP characteristic values for each of the subsequent amplitude values; and
control a user interface to display updates to the graph as additional ECAP characteristic values are determined from delivered stimulation pulses of the plurality of stimulation pulses.

5. The system of any of claims 1 through 4, wherein the processing circuitry (210) is configured to:
control the stimulation generation circuitry to interleave the delivery the plurality of stimulation pulses from a first electrode combination and additional stimulation pulses from a second electrode combination;
receive, for each stimulation pulse of the plurality of stimulation pulses and the additional stimulation pulses, (ECAP signal information;
determine, from the ECAP signal information for each stimulation pulse, ECAP characteristic values for each of the ECAP signals;
control a user interface to display a graph of the ECAP characteristic values for each of the subsequent amplitude values for each of the plurality of stimulation pulses from the first electrode combination and the additional stimulation pulses from the second electrode combination; and
determine, based on the ECAP characteristic values and during the delivery of the stimulation pulses and additional stimulation pulses with iteratively increasing amplitude values, at least one threshold from which one or more stimulation parameter values for subsequent stimulation pulses are determined for each of the first electrode combination and the second electrode combination.

6. The system of any of claims 1 through 5, wherein the plurality of stimulation pulses begin at an initial amplitude value less than the maximum amplitude value, and wherein the processing circuitry (210) is configured to iteratively increase a subsequent amplitude value of a next stimulation pulse of the plurality of stimulation pulses up to the maximum amplitude value.

7. The system of any of claims 1 through 6, wherein the at least one threshold comprises a perception threshold.

8. The system of any of claims 1 through 7, wherein the processing circuitry (210) is configured to terminate the delivery of the plurality of stimulation pulses in response to determining the at least one threshold.

9. The system of any of claims 1 through 8, wherein the processing circuitry (210) is further configured to control stimulation circuitry to generate and deliver stimulation pulses at a predetermined percentage of the at least one threshold.

10. The system of any of claims 1 through 9, wherein the one or more stimulation parameter values comprises an amplitude, a pulse width, a pulse rate, or a duty cycle.

11. The system of any of claims 1 through 10, wherein the processing circuitry (210) is configured to determine the at least one threshold at least in part on a curvature of an inflection region of a growth curve corresponding to the ECAP characteristic values.

12. The system of any of claims 1 through 11, wherein the ECAP characteristic values are indicative of a direct measurement between an N1 peak and a P2 peak of the ECAP signal information for each ECAP signal of the ECAP signals.

13. The system of any of claims 1 through 12, further comprising an implantable medical device comprising at least a portion of the processing circuitry (210).

14. The system of any of claims 1 through 13, further comprising an external programmer comprising at least a portion of the processing circuitry (210) and a user interface configured to receive user input identifying the maximum amplitude value.

15. A computer-readable medium comprising instructions that, when executed, control processing circuitry (210) to:
receive a maximum amplitude value for a plurality of stimulation pulses;
control stimulation generation circuitry to begin delivery of the plurality of stimulation pulses at an initial amplitude value less than the maximum amplitude value and iteratively increase a subsequent amplitude value of a next stimulation pulse of the plurality of stimulation pulses up to the maximum amplitude value;
receive, for each stimulation pulse of the plurality of stimulation pulses, evoked compound action potential (ECAP) signal information, wherein the ECAP signal information comprises ECAP signals sensed by sensing circuitry;
determine, from the ECAP signal information for each stimulation pulse, ECAP characteristic values for each of the ECAP signals; and
determine, based on the ECAP characteristic values and during the delivery of the stimulation pulses with iteratively increasing amplitude values, at least one threshold from which one or more stimulation parameter values for subsequent stimulation pulses are determined.

## Patentansprüche

1. System, umfassend:
eine Verarbeitungsschaltung (210), die konfiguriert ist zum:
Empfangen eines maximalen Amplitudenwerts für eine Vielzahl von Stimulationsimpulsen;
Steuern einer Stimulationserzeugungsschaltung, um mit einer Abgabe der Vielzahl von Stimulationsimpulsen bei einem anfänglichen Amplitudenwert, der kleiner als der maximale Amplitudenwert ist, zu beginnen und einen nachfolgenden Amplitudenwert eines nächsten Stimulationsimpulses der Vielzahl von Stimulationsimpulsen bis auf den maximalen Amplitudenwert iterativ zu erhöhen;
Empfangen, für jeden Stimulationspuls der Vielzahl von Stimulationspulsen, von Signalinformationen eines evozierten Summenaktionspotenzials (ECAP-Signalinformationen), wobei die ECAP-Signalinformationen ECAP-Signale umfassen, die durch eine Erfassungsschaltung erfasst werden;
Bestimmen, aus den ECAP-Signalinformationen für jeden Stimulationsimpuls, von ECAP-Kennwerten für jedes der ECAP-Signale; und
Bestimmen, basierend auf den ECAP-Kennwerten und während der Abgabe der Stimulationsimpulse mit sich iterativ erhöhenden Amplitudenwerten, mindestens einer Schwelle, aus der ein oder mehrere Stimulationsparameterwerte für nachfolgende Stimulationsimpulse bestimmt werden.

2. System nach Anspruch 1, wobei die Verarbeitungsschaltung (210) konfiguriert ist zum:
Empfangen des maximalen Amplitudenwerts über eine Benutzerschnittstelle;
Steuern der Benutzerschnittstelle, um eine Darstellung eines Bereichs von Amplitudenwerten für die Vielzahl von Stimulationsimpulsen anzuzeigen, die während einer Kalibrierungsroutine abgebbar sind;
Empfangen, über die Benutzerschnittstelle, einer Benutzereingabe, die den maximalen Amplitudenwert spezifiziert; und
Steuern der Benutzerschnittstelle, um eine Darstellung der mindestens einen Schwelle anzuzeigen.

3. System nach einem der Ansprüche 1 und 2, wobei die Verarbeitungsschaltung (210) konfiguriert ist, um die mindestens eine Schwelle auf der Darstellung des Bereichs von Amplitudenwerten zu präsentieren.

4. System nach einem der Ansprüche 1 bis einschließlich 3, wobei die Verarbeitungsschaltung (210) konfiguriert ist zum:
Erzeugen eines Graphen der ECAP-Kennwerte für jeden der nachfolgenden Amplitudenwerte; und
Steuern einer Benutzerschnittstelle, um Aktualisierungen des Graphen anzuzeigen, während zusätzliche ECAP-Kennwerte aus abgegebenen Stimulationsimpulsen der Vielzahl von Stimulationsimpulsen bestimmt werden.

5. System nach einem der Ansprüche 1 bis einschließlich 4, wobei die Verarbeitungsschaltung (210) konfiguriert ist zum:
Steuern der Stimulationserzeugungsschaltung, um die Abgabe der Vielzahl von Stimulationsimpulsen von einer ersten Elektrodenkombination und zusätzlicher Stimulationsimpulse von einer zweiten Elektrodenkombination zu verschachteln;
Empfangen, für jeden Stimulationsimpuls der Vielzahl von Stimulationsimpulsen und der zusätzlichen Stimulationsimpulse, (ECAP-Signalinformationen;
Bestimmen, aus den ECAP-Signalinformationen für jeden Stimulationsimpuls, von ECAP-Kennwerten für jedes der ECAP-Signale;
Steuern einer Benutzerschnittstelle, um einen Graphen der ECAP-Kennwerte für jeden der nachfolgenden Amplitudenwerte für jeden der Vielzahl von Stimulationsimpulsen von der ersten Elektrodenkombination und der zusätzlichen Stimulationsimpulse von der zweiten Elektrodenkombination anzuzeigen; und
Bestimmen, basierend auf den ECAP-Kennwerten und während der Abgabe der Stimulationsimpulse und zusätzlicher Stimulationsimpulse mit sich iterativ erhöhenden Amplitudenwerten, mindestens einer Schwelle, aus der ein oder mehrere Stimulationsparameterwerte für nachfolgende Stimulationsimpulse für jede der ersten Elektrodenkombination und der zweiten Elektrodenkombination bestimmt werden.

6. System nach einem der Ansprüche 1 bis einschließlich 5, wobei die Vielzahl von Stimulationsimpulsen bei einem anfänglichen Amplitudenwert beginnt, der kleiner als der maximale Amplitudenwert ist, und wobei die Verarbeitungsschaltung (210) konfiguriert ist, um einen nachfolgenden Amplitudenwert eines nächsten Stimulationsimpulses der Vielzahl von Stimulationsimpulsen bis zu dem maximalen Amplitudenwert iterativ zu erhöhen.

7. System nach einem der Ansprüche 1 bis einschließlich 6, wobei die mindestens eine Schwelle eine Wahrnehmungsschwelle umfasst.

8. System nach einem der Ansprüche 1 bis einschließlich 7, wobei die Verarbeitungsschaltung (210) konfiguriert ist, um die Abgabe der Vielzahl von Stimulationsimpulsen als Reaktion auf das Bestimmen der mindestens einen Schwelle zu beenden.

9. System nach einem der Ansprüche 1 bis einschließlich 8, wobei die Verarbeitungsschaltung (210) ferner konfiguriert ist, um eine Stimulationsschaltung zu steuern, um Stimulationsimpulse mit einem vorbestimmten Prozentsatz der mindestens einen Schwelle zu erzeugen und abzugeben.

10. System nach einem der Ansprüche 1 bis einschließlich 9, wobei der eine oder die mehreren Stimulationsparameterwerte eine Amplitude, eine Pulsbreite, eine Pulsrate oder ein Tastverhältnis umfasst.

11. System nach einem der Ansprüche 1 bis einschließlich 10, wobei die Verarbeitungsschaltung (210) konfiguriert ist, um die mindestens einen Schwelle mindestens teilweise basierend auf einer Krümmung eines Wendebereichs einer Wachstumskurve zu bestimmen, die den ECAP-Kennwerten entspricht.

12. System nach einem der Ansprüche 1 bis einschließlich 11, wobei die ECAP-Kennwerte eine direkte Messung zwischen einem N1-Peak und einem P2-Peak der ECAP-Signalinformationen für jedes ECAP-Signal der ECAP-Signale anzeigen.

13. System nach einem der Ansprüche 1 bis einschließlich 12, ferner umfassend eine implantierbare medizinische Vorrichtung, umfassend mindestens einen Abschnitt der Verarbeitungsschaltung (210).

14. System nach einem der Ansprüche 1 bis einschließlich 13, ferner umfassend eine externe Programmiereinrichtung, umfassend mindestens einen Abschnitt der Verarbeitungsschaltung (210) und eine Benutzerschnittstelle, die konfiguriert ist, um eine Benutzereingabe zu empfangen, die den maximalen Amplitudenwert identifiziert.

15. Computerlesbares Medium, umfassend Anweisungen, die, wenn sie ausgeführt werden, die Verarbeitungsschaltung (210) steuern zum:
Empfangen eines maximalen Amplitudenwerts für eine Vielzahl von Stimulationsimpulsen;
Steuern einer Stimulationserzeugungsschaltung, um mit der Abgabe der Vielzahl von Stimulationsimpulsen bei einem anfänglichen Amplitudenwert, der kleiner als der maximale Amplitudenwert ist, zu beginnen und einen nachfolgenden Amplitudenwert eines nächsten Stimulationsimpulses der Vielzahl von Stimulationsimpulsen bis auf den maximalen Amplitudenwert iterativ zu erhöhen;
Empfangen, für jeden Stimulationspuls der Vielzahl von Stimulationspulsen, von Signalinformationen des evozierten Summenaktionspotenzials (ECAP-Signalinformationen), wobei die ECAP-Signalinformationen ECAP-Signale umfassen, die durch eine Erfassungsschaltung erfasst werden;
Bestimmen, aus den ECAP-Signalinformationen für jeden Stimulationsimpuls, von ECAP-Kennwerten für jedes der ECAP-Signale; und
Bestimmen, basierend auf den ECAP-Kennwerten und während der Abgabe der Stimulationsimpulse mit sich iterativ erhöhenden Amplitudenwerten, mindestens einer Schwelle, aus der ein oder mehrere Stimulationsparameterwerte für nachfolgende Stimulationsimpulse bestimmt werden.

## Revendications

1. Système comprenant :
des circuits de traitement (210) configurés pour :
recevoir une valeur d'amplitude maximale pour une pluralité d'impulsions de stimulation ;
commander les circuits de génération de stimulation pour commencer l'administration de la pluralité d'impulsions de stimulation à une valeur d'amplitude initiale inférieure à la valeur d'amplitude maximale et augmenter de manière itérative une valeur d'amplitude subséquente d'une impulsion de stimulation suivante de la pluralité d'impulsions de stimulation jusqu'à la valeur d'amplitude maximale ;
recevoir, pour chaque impulsion de stimulation de la pluralité d'impulsions de stimulation, des informations de signal de potentiel d'action composé évoqué (ECAP), dans lequel les informations de signal ECAP comprennent des signaux ECAP détectés par les circuits de détection ;
déterminer, à partir des informations de signal ECAP pour chaque impulsion de stimulation, des valeurs caractéristiques d'ECAP pour chacun des signaux ECAP ; et
déterminer, sur la base des valeurs caractéristiques d'ECAP et pendant l'administration des impulsions de stimulation avec des valeurs d'amplitude augmentant de manière itérative, au moins un seuil à partir duquel une ou plusieurs valeurs de paramètre de stimulation pour des impulsions de stimulation subséquentes sont déterminées.

2. Système selon la revendication 1, dans lequel l'ensemble de circuits de traitement (210) est configuré pour :
recevoir la valeur d'amplitude maximale par le biais d'une interface utilisateur ;
commander l'interface utilisateur pour afficher une représentation d'une plage de valeurs d'amplitude pour la pluralité d'impulsions de stimulation pouvant être administrées pendant une routine d'étalonnage ;
recevoir, par le biais de l'interface utilisateur, une entrée utilisateur spécifiant la valeur d'amplitude maximale ; et
commander l'interface utilisateur pour afficher une représentation de l'au moins un seuil.

3. Système selon l'une quelconque des revendications 1 et 2, dans lequel les circuits de traitement (210) sont configurés pour présenter l'au moins un seuil sur la représentation de la plage de valeurs d'amplitude.

4. Système selon l'une quelconque des revendications 1 à 3, dans lequel le circuit de traitement (210) est configuré pour :
générer un graphique des valeurs caractéristiques d'ECAP pour chacune des valeurs d'amplitude subséquentes ; et
commander une interface utilisateur pour afficher des mises à jour du graphique à mesure que des valeurs de caractéristique d'ECAP supplémentaires sont déterminées à partir d'impulsions de stimulation administrées de la pluralité d'impulsions de stimulation.

5. Système selon l'une quelconque des revendications 1 à 4, dans lequel les circuits de traitement (210) sont configurés pour :
commander les circuits de génération de stimulation pour entrelacer l'administration de la pluralité d'impulsions de stimulation à partir d'une première combinaison d'électrodes et des impulsions de stimulation supplémentaires à partir d'une seconde combinaison d'électrodes ;
recevoir, pour chaque impulsion de stimulation de la pluralité d'impulsions de stimulation et des impulsions de stimulation supplémentaires, des informations de signal ECAP ;
déterminer, à partir des informations de signal ECAP pour chaque impulsion de stimulation, des valeurs caractéristiques d'ECAP pour chacun des signaux ECAP ;
commander une interface utilisateur pour afficher un graphique des valeurs caractéristiques d'ECAP pour chacune des valeurs d'amplitude subséquentes pour chaque impulsion de la pluralité d'impulsions de stimulation provenant de la première combinaison d'électrodes et des impulsions de stimulation supplémentaires provenant de la seconde combinaison d'électrodes ; et
déterminer, sur la base des valeurs caractéristiques d'ECAP et pendant l'administration des impulsions de stimulation et des impulsions de stimulation supplémentaires avec des valeurs d'amplitude augmentant de manière itérative, au moins un seuil à partir duquel une ou plusieurs valeurs de paramètre de stimulation pour des impulsions de stimulation subséquentes sont déterminées pour chacune de la première combinaison d'électrodes et de la seconde combinaison d'électrodes.

6. Système selon l'une quelconque des revendications 1 à 5, dans lequel la pluralité d'impulsions de stimulation commence à une valeur d'amplitude initiale inférieure à la valeur d'amplitude maximale, et dans lequel les circuits de traitement (210) sont configurés pour augmenter de manière itérative une valeur d'amplitude subséquente d'une impulsion de stimulation suivante de la pluralité d'impulsions de stimulation jusqu'à la valeur d'amplitude maximale.

7. Système selon l'une quelconque des revendications 1 à 6, dans lequel l'au moins un seuil comprend un seuil de perception.

8. Système selon l'une quelconque des revendications 1 à 7, dans lequel les circuits de traitement (210) sont configurés pour mettre fin à l'administration de la pluralité d'impulsions de stimulation en réponse à la détermination de l'au moins un seuil.

9. Système selon l'une quelconque des revendications 1 à 8, dans lequel les circuits de traitement (210) sont en outre configurés pour commander des circuits de stimulation pour générer et administrer des impulsions de stimulation à un pourcentage prédéterminé de l'au moins un seuil.

10. Système selon l'une quelconque des revendications 1 à 9, dans lequel les une ou plusieurs valeurs de paramètre de stimulation comprennent une amplitude, une largeur d'impulsion, une fréquence d'impulsion ou un rapport cyclique.

11. Système selon l'une quelconque des revendications 1 à 10, dans lequel les circuits de traitement (210) sont configurés pour déterminer l'au moins un seuil au moins en partie sur une courbure d'une région d'inflexion d'une courbe de croissance correspondant aux valeurs caractéristiques d'ECAP.

12. Système selon l'une quelconque des revendications 1 à 11, dans lequel les valeurs caractéristiques d'ECAP sont révélatrices d'une mesure directe entre un pic N1 et un pic P2 des informations de signal d'ECAP pour chaque signal ECAP des signaux ECAP.

13. Système selon l'une quelconque des revendications 1 à 12, comprenant en outre un dispositif médical implantable comprenant au moins une partie des circuits de traitement (210).

14. Système selon l'une quelconque des revendications 1 à 13, comprenant en outre un programmateur externe comprenant au moins une partie des circuits de traitement (210) et une interface utilisateur configurée pour recevoir une entrée utilisateur identifiant la valeur d'amplitude maximale.

15. Support lisible par ordinateur comprenant des instructions qui, lorsqu'elles sont exécutées, commandent les circuits de traitement (210) pour :
recevoir une valeur d'amplitude maximale pour une pluralité d'impulsions de stimulation ;
commander les circuits de génération de stimulation pour commencer l'administration de la pluralité d'impulsions de stimulation à une valeur d'amplitude initiale inférieure à la valeur d'amplitude maximale et augmenter de manière itérative une valeur d'amplitude subséquente d'une impulsion de stimulation suivante de la pluralité d'impulsions de stimulation jusqu'à la valeur d'amplitude maximale ;
recevoir, pour chaque impulsion de stimulation de la pluralité d'impulsions de stimulation, des informations de signal de potentiel d'action composé évoqué (ECAP), dans lequel les informations de signal ECAP comprennent des signaux ECAP détectés par les circuits de détection ;
déterminer, à partir des informations de signal ECAP pour chaque impulsion de stimulation, des valeurs caractéristiques d'ECAP pour chacun des signaux ECAP ; et
déterminer, sur la base des valeurs caractéristiques d'ECAP et pendant l'administration des impulsions de stimulation avec des valeurs d'amplitude augmentant de manière itérative, au moins un seuil à partir duquel une ou plusieurs valeurs de paramètre de stimulation pour des impulsions de stimulation subséquentes sont déterminées.
